(19) Europäisches Patentamt
European Patent Office
Office européen des brevets

(11) EP 4 810 117 A2

(12) # EUROPEAN PATENT APPLICATION

(43) Date of publication:
**23.09.2026 Bulletin 2026/39**

(21) Application number: **26195668.4**

(22) Date of filing: **30.07.2021**

(51) International Patent Classification (IPC):
***A61P 25/28*** *(2006.01)*

(52) Cooperative Patent Classification (CPC):
(C-Sets available)
**A61P 3/00; A61K 31/445; A61K 31/7016; A61P 25/28** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.07.2020 EP 20188648**

(62) Document number(s) of the earlier application(s) in accordance with Art. 76 EPC:
**21755926.9 / 4 188 371**

(71) Applicants:
- **Beyond Batten Disease Foundation Austin TX 78763 (US)**
- **Baylor College of Medicine Houston, TX 77030 (US)**
- **University College Cardiff Consultants Limited Cardiff, South Wales CF24 ODE (GB)**

(72) Inventors:
- **KERKOVICH, Danielle AUSTIN, TX78763 (US)**
- **WALLER-EVANS, Helen CARDIFF, CF10 3AT (GB)**
- **LLOYD- EVANS, Emyr CARDIFF, CF10 3AX (GB)**
- **AMAWI, Abdallah HOUSTON, TX77030-341 (US)**
- **LOFTI, Parisa HOUSTON, TX77030-341 (US)**
- **SARDIELLO, Marco SAINT-LOUIS, MO 63110 (US)**

(74) Representative: **Santarelli Tour Trinity 1 bis Place de la Défense 92400 Courbevoie (FR)**

Remarks:
This application was filed on 30-07-2026 as a divisional application to the application mentioned under INID code 62.

(54) **PRODUCTS FOR TREATING THE JNCL DISEASE**

(57) The present invention is based on the finding that parenteral administration of Trehalose leads to surprisingly high amounts of Trehalose that reach and accumulate in the brain compared to other routes, where it inhibits neuronal cell death and reduces neuroinflammation in a mouse model of a lysosomal disease (CLN3). It is also based on the finding that Miglustat has a very pronounced beneficial effect on neuronal cell death, on its own, when orally administered at low and high doses in this mouse model and that this strong effect is even more pronounced in the presence of Trehalose. The present invention therefore relates to a product containing Miglustat alone, or to a combination product containing parenteral Trehalose and oral Miglustat, for separate administration, for treating lysosomal diseases such as the CLN3 disease. In particular, the invention concerns a combination product comprising Trehalose and Miglustat, or any pharmaceutically acceptable salt thereof, for simultaneous, separated, or staggered use for treating a lysosomal storage disorder or a disorder characterized by lysosomal dysfunction in a subject in need thereof, wherein Trehalose is administered parenterally and Miglustat is administered orally.

EP 4 810 117 A2

(52) Cooperative Patent Classification (CPC): (Cont.)

C-Sets
**A61K 31/445, A61K 2300/00;**
**A61K 31/7016, A61K 2300/00**

## Description

### SUMMARY OF THE INVENTION

The present invention is based on the finding that parenteral administration of Trehalose leads to surprisingly high amounts of Trehalose that reach and accumulate in the brain compared to other routes, where it inhibits neuronal cell death and reduces neuroinflammation in a mouse model of a lysosomal disease (CLN3). It is also based on the finding that the trehalase (the enzyme responsible for the metabolism of trehalose) inhibitor Miglustat has a more pronounced beneficial effect on neuronal cell death, on its own, when orally administered at low and high doses in this mouse model and that the effect is even more pronounced in the presence of Trehalose. The present invention therefore relates to a combination product containing parenteral Trehalose and oral Miglustat, for separate administration, for treating lysosomal diseases such as the CLN3 form of the Batten disease.

### BACKGROUND OF THE INVENTION

Lysosomes are membrane-bound cell organelles central to degradation processes in animal cells. Extracellular materials such as microorganisms taken up by phagocytosis, macromolecules by endocytosis, and unwanted cell organelles, fuse with lysosomes and are broken down to their basic molecules. Thus, lysosomes are the recycling units of a cell. Lysosomes are also responsible for cellular homeostasis for their role in secretion, plasma membrane repair, cell signaling, and energy metabolism.

The essential role of lysosomes in cellular degradation processes puts these organelles at the crossroads of several cellular processes, with significant implications for health and disease. Defects in one of 60 lysosomal enzymes, transmembrane proteins or other components of this organelle, prevent the breakdown of target molecules, and are responsible for more than 60 different human genetic diseases, which are collectively known as lysosomal storage disorders. The large number and variety of human pathological conditions that are characterized, if not caused by aberrant lysosomal functions, underscores the critical importance of the autophagy-lysosome pathways to cellular metabolism. In these diseases as well as diseases characterized by lysosomal dysfunction, undegraded materials accumulate within the lysosomes, contributing to the presence or severity of disease ranging from lysosomal storage disorders to neurodegenerative diseases, to cancer, to cardiovascular disease.

For instance, the neuronal ceroid lipofuscinoses (NCLs), lysosomal storage disorders also known as Batten disease, are a group of neurodegenerative disorders considered the most common of the neurogenetic storage diseases, with a prevalence of 1 in 12,500 in some populations. There are currently no cures or approved treatments for any of the 14 forms of Batten disease.

CLN3 disease is an ultra-rare, genetic, lysosomal storage disease that primarily affects the nervous system and is fatal. Children with the CLN3 form of the Batten disease develop normally, even excelling in school until ages 4-7 years, when progressive vision loss becomes noticeable (Aberg et al., 2011; Bozorg et al., 2009). Concomitantly, or shortly thereafter, parents report personality changes and behavioral issues. Typically, within 2-3 years after symptom onset, total vision loss occurs and seizures begin. This is followed by declining speech, progressive loss of motor coordination and cardiac involvement. Psychosis, hallucinations and/or dementia can appear anytime during the disease. Eventually, children become wheelchair-bound, then bedridden and die in their late teens or early twenties (Ostergaard, 2016).

The pathological hallmark of the CLN3 form of the Batten disease is the accumulation of incompletely digested material which causes an autophagic block leading to progressive axonal degeneration and neuron loss which is amplified by chronic neuroinflammation (Nixon and Yang, 2012). Evidence from animal models and cell culture from both animal and CLN3 patient cells demonstrates that accumulation of pathologic material, neuron loss (apoptosis) and neuroinflammation are inhibited by the clearance of protein and lipid aggregates (Palmieri et al., 2017a; Palmieri et al., 2017b; Settembre et al., 2011). Additionally, recent preclinical evidence suggests that these defects in autophagy may underlie the enhanced levels of $\alpha$-synuclein oligomers, gangliosides GM1, GM2, and GM3 as well as reduced levels of sphingomyelin and autophagy observed in cellular models of CLN3 disease (Kang et al., 2014).

A key cellular homeostatic pathway implicated in the CLN3 form of the Batten disease (Cao et al., 2006; Radke et al., 2018) and a myriad of other lysosomal storage disorders is autophagy. Autophagy is vital for the maintenance of energy and tissue homeostasis by degrading damaged or excess intracellular components such as aggregation-prone proteins, lipids, and organelles, and recycling the breakdown products. The nervous system appears to be particularly susceptible to the effects of defective lysosomal autophagy, likely due to a combination of the long-lived nature of post-mitotic neurons placing particular stress on protein-clearing processes, the extreme polarization of many neurons, and the high metabolic requirements of neurons leading to higher levels of oxidative damage in lysosomes via the Fenton reaction. Autophagy deficiency in neurons and the subsequent failure to prevent accumulation of abnormal proteins leads to neurodegeneration (Nikoletopoulou et al., 2015), which is a likely mechanism underlying the neuropathology observed in the CLN3 form of the Batten disease.

While the primary cellular function of the CLN3 protein is still not clear, CLN3 deficiency has been linked to defects in the maturation and fusion of autophagosomes and endolysosomal compartments, lysosomal pH, and the motility of late endosomes and lysosomes, most likely through interactions with Hook1 and microtubule motor protein complexes (Fossale et al., 2004; Luiro et al., 2004; Cao et al., 2006; Uusi-Rauva et al., 2008; Uusi-Rauva et al., 2012), proliferation and apoptosis (Cotman and Staropoli, 2012; Cárcel-Trullols et al., 2015).

The Batten disease, and lysosomal storage diseases in general, are known to involve degeneration of neuronal brain cells. It is therefore of primary importance that their proposed treatment can pass the blood-brain barrier and effectively reach the brain, where it should remain at an effective concentration for a sufficient time to achieve a significant therapeutic effect.

It has been previously proposed to treat patients suffering from lysosomal diseases such as the San Filippo disease with oral uptake of Trehalose (Thesis of V.Mauri et al., 2014, WO 2017/185010, Palmieri et al). Yet, in some of these studies, Trehalose was shown not to reach the brain, when orally administered (Mauri's thesis). According to Mauri's thesis, this could be due to the fact that Trehalose is hydrolyzed by the intrinsic enzyme Trehalase at the epithelial brush border in the small intestine what causes only a small fraction of any orally administered dose to reach blood stream or tissues (see also in Cendret et al.; Tanaka et al., 2004).

Other studies have disclosed that Trehalose has limited access to the brain, as its passage would be blocked by the blood-brain barrier (Lee et al, 2018). To counteract this, some authors have proposed to use exceedingly high and continuous oral intake of Trehalose, in order to achieve the desired concentration in the brain tissue (Mauri's thesis, WO2017/136922). However, high dose of Trehalose was shown to induce cytotoxic effects (Khalifeh et al., 2019). Also, it was proposed to combine oral Trehalose with a Trehalase inhibitor such as Miglustat (WO2017/185010), or to administer Trehalose directly in the brain (Mauri's thesis).

Although *in vitro* models have been useful to demonstrate a possible mode of action for Trehalose's efficacy *in vivo*, given Trehalose's typical post-ingestion digestive and metabolic fate, *in vivo* data are needed to demonstrate intact Trehalose absorption, distribution, metabolism and elimination after its administration and to identify adequate and clinically relevant routes of administration. The present invention solved this need. As a matter of fact, the present inventors have studied the concentration of circulating Trehalose and assessed which location (brain or periphery) it can reach effectively, depending on its route of administration. By doing so, they demonstrated that, contrary to what was suggested in the prior art, even when parenterally (and not only intracerebrally) administered, Trehalose can be found in relatively high levels in the brain, where it has an effect on neuronal cell death, neuroinflammation and microglial activation. More importantly, they show that the brain level of Trehalose is higher when Trehalose is parenterally administered, as compared with an oral administration. This surprising finding enables to consider using this new route of administration for treating lysosomal diseases, instead of the usual oral intake. This will allow to lower the dose of injected Trehalose, thereby avoiding any side effect that could be attributed to the ingestion of high doses of Trehalose (Khalifeh et al., 2019).

In addition, the authors demonstrated that oral miglustat is able to increase the concentration of parenterally-administered trehalose in the blood, the liver and in the brain, hence reinforcing the ADME properties of Trehalose. They also herein show that oral miglustat, even at low doses, is able to reduce neuronal cell death, microglial activation and neuroinflammation on its own, in a CLN3 animal model.

Based on these surprising results, the inventors now propose two novel and optimized therapeutic regimen for treating disorders involving abnormal lysosomal storage, and the CLN3 form of Batten disease in particular:

- The first one requires parenteral administration of Trehalose and oral administration of Miglustat,
- The second one involving oral administration of Miglustat (or of other inhibitors of the glucosylceramide synthase) alone or in combination with parenteral Trehalose.

These regimen are likely to provide a higher therapeutic efficacy in the treatment of disorders involving abnormal lysosomal storage, and the CLN3 form of Batten disease in particular, as those proposed in the art (involving mainly oral Trehalose).

## DETAILED DESCRIPTION OF THE INVENTION

Trehalose, a known pharmacological inducer of autophagy and lysosomal pathways, increases the life span and normalizes behavioral and neuropathological aggregates in animal models of Parkinson's disease (Khalifeh et al., 2019), Huntington's disease (Sarkar and Rubinsztein, 2008), and Alzheimer disease (Du et al., 2013; Tien et al., 2016; Portbury et al., 2017). The mechanisms for Trehalose-induced autophagy induction in CLN3 were previously unknown until BBDF-sponsored studies in a mouse model of CLN3 disease and in cells from patients and mice with CLN3 disease. These studies demonstrated that Trehalose enhances the clearance of proteolipid aggregates *via* modulation of transcription factor EB (TFEB), a master regulator of lysosomal pathways, governing lysosomal biogenesis and metabolism (Sardiello et al., 2009; Palmieri et al., 2017a; Palmieri et al., 2017b), autophagy (Settembre et al., 2011), lysosomal exocytosis

(Medina et al., 2011) and proteostasis (Song et al., 2013).

Trehalose may further enhance autophagy through inhibition of the SLCA2 family of glucose receptors, resulting in activation of an AMPK-dependent autophagy pathway (Dehay et al., 2010; Uchida et al., 2014; DeBosch et al., 2016). By inducing autophagy, Trehalose protects cells against pro-apoptotic insults (Sarkar et al., 2007) and may act as a chemical chaperone to prevent protein misfolding contributing to aggregate formation (Perucho et al., 2012; Sarkar et al., 2014; Zhang et al., 2014).

The present inventors have shown that, contrary to what was suggested in the prior art, parenterally administered Trehalose can be found in relatively high levels in brain, where it has an effect on neuronal cell death, neuroinflammation and microglial activation. More importantly, they show that the brain level of Trehalose is higher when Trehalose is parenterally administered, as compared with an oral administration. This surprising finding enables to consider using this new route of administration for treating lysosomal diseases, instead of the usual oral intake.

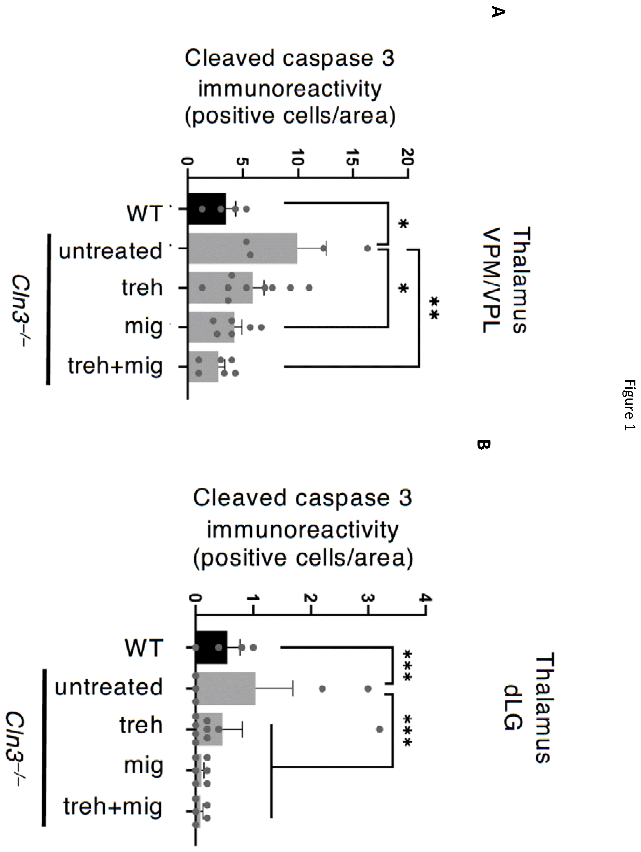

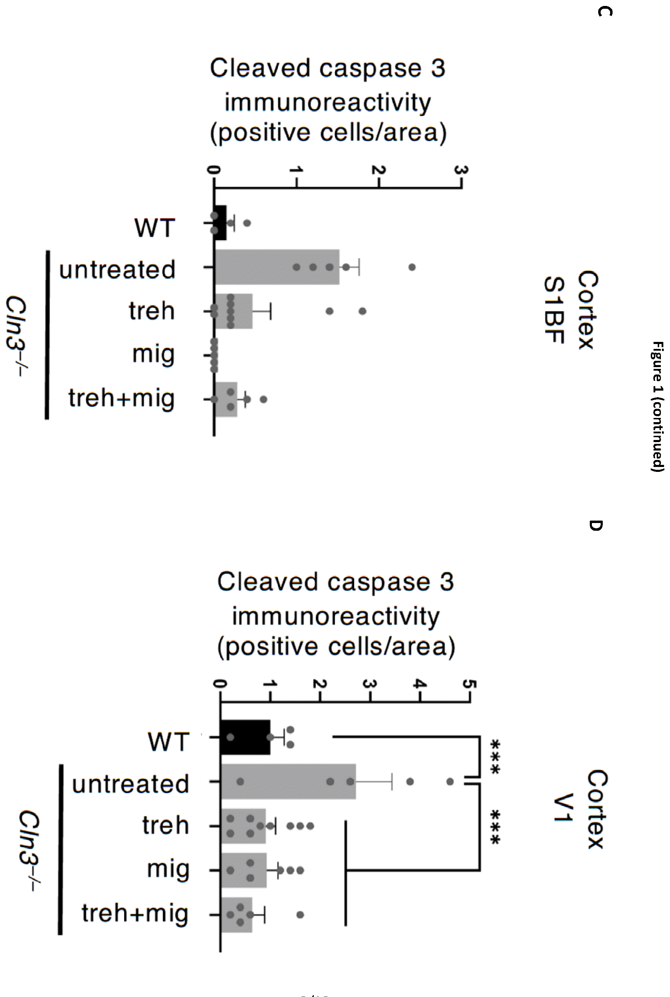

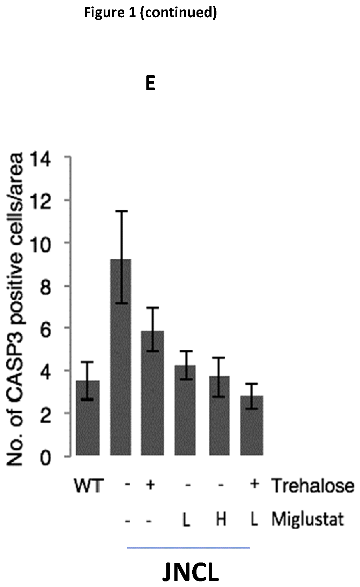

The present inventors have moreover investigated the effect of the Trehalase inhibitor Miglustat *in vivo* in a mouse model of Batten disease. They have shown that, surprisingly, Miglustat has also a strong effect on its own on neuronal cell death, neuroinflammation and microglial activation, even at low dose (Figures 1-3). As explained below, this effect could be linked to the regulatory activity of Miglustat on the glycosphingolipid synthesis, more precisely on its ability to reduce abnormal ganglioside accumulation in lysosomes from CLN3 deficient cells.

The present inventors also show that the level of Trehalose in the brain is much higher when Miglustat and Trehalose are separately administered to a subject. This could be due to a stabilizing effect of Miglustat on Trehalose, in the brain, or an inhibition of trehalase, the enzyme degrading Trehalose.

In view of i) the activation of autophagy by Trehalose, ii) the ability of Miglustat to reduce both ganglioside accumulation and inflammation, and iii) the potential increased exposure of Trehalose *via* trehalase inhibition or direct stabilization (by Miglustat), all these results suggest that the combination of Trehalose and Miglustat or the use of Miglustat alone has the potential to slow the progression of CLN3 disease.

Combination of Trehalose (IV) and Miglustat (oral)

In a first aspect, the present inventors therefore propose a novel and optimized therapeutic regimen using parenteral administration of Trehalose and oral administration of Miglustat. This particular regimen is likely to display a higher therapeutic efficacy in the treatment of disorders involving abnormal lysosomal storage, and the CLN3 for of Batten disease, in particular, because parenterally-administrated Trehalose accumulates in the brain of mice that are treated with this combination product. This is all the more surprising as it was thought that Trehalose is hydrolyzed by the intrinsic enzyme trehalase at the epithelial brush border in the small intestine (Cendret et al.; Tanaka et al., 2004; Thesis of Mauri et al.), what causes only a small fraction of any enterally administered dose to reach blood stream, neuronal, brain or muscle tissues.

In this first aspect, the present invention relates to a combination product comprising Trehalose and Miglustat, or any pharmaceutically acceptable salt thereof, for simultaneous, separated, or staggered use for treating a lysosomal storage disorder or a disorder characterized by lysosomal dysfunction in a subject in need thereof, wherein Trehalose is administered parenterally and Miglustat is administered orally.

The present invention also discloses a method for treating or alleviating a lysosomal storage disorder, or a disorder characterized by lysosomal dysfunction, or at least one symptom associated therewith, in a human subject in need thereof, said method comprising parenterally administering to said subject a therapeutically effective amount of Trehalose or a pharmaceutical formulation comprising a therapeutically effective amount of Trehalose, and orally administering a therapeutically amount of Miglustat.

The term "Trehalose" as used herein refers to the form of the Trehalose compound *per se*, as well as any other form such as a salt, polymorph, enantiomer, stereoisomer, conformer, solvate, ester, amide, prodrug, analog, derivative, or the like, provided that said salt, polymorph, ester, enantiomer, stereoisomer, conformer, solvate, amide, prodrug, analog, or derivative is suitable pharmacologically of a trehalose analog capable of inhibiting the AKTenzyme. Any crystalline form of Trehalose is herewith encompassed, notably those disclosed in US20010033888. The crystal structure of the rhomboid Trehalose dihydrate has been published by Taga et al. in 1972. Anhydrous form of Trehalose can also be used in the composition of the invention. In the composition product of the invention, the Trehalose can be a Trehalose analog. The Trehalose analog can be selected from lentzTrehalose A, lentzTrehalose B, and lentzTrehalose C (Wada et al).

Trehalose, also known as mycose or tremalose, is a stable, non-reducing disaccharide with two glucose molecules linked in a 1,1 configuration. The structure of Trehalose is diagrammed below (Table I). Trehalose has protein-stabilizing properties, and is extensively used in many applications as a stabilizer of frozen food, in freeze-drying of biological systems and cells, as a stabilizer of therapeutic parenteral proteins, and as an excipient in tablets and IV solutions. Trehalose is recognized as a GRAS (Generally Regarded as Safe) food ingredient by the FDA and is listed on the USP-NF (United States Pharmacopoeia National Formulary), EP (European Pharmacopoeia) and JP (Japanese Pharmacopoeia). The safety and toxicity of Trehalose has been extensively investigated, and the substance was found

to be safe when administered both orally and intravenously, in doses that are substantially higher than the intended therapeutic dose.

Preferably, Trehalose is substantially free of contaminants resulting from isolation and purification process of Trehalose. Trehalose may be isolated by extraction from dry yeast or the like; by enzymatic production and isolation; and by the culturing of microorganisms. As such, Trehalose is preferably substantially free of such contaminants as enzymes, organic solvents such as ammonium, acetonitrile, acetamide, alcohol (e.g., methanol, ethanol, or isopropanol), TFA, ether, or other contaminants used in a process for preparing and purifying trehalose. The term "substantially" free of contaminants may refer to Trehalose having a contaminant content of preferably less than 0.5%, less than 0.3%, less than 0.25%, less than 0.1%, less than 0.05%, less than 0.04%, less than 0.03%, less than 0.02%, less than 0.01%, less than 0.005%, less than 0.003%, or less than 0.001% of the total weight of the Trehalose. Methods of determining the content of contaminants is known in the art and may be determined by conventional methods such as gas chromatography. Preferably, the residual solvents in the purified Trehalose of the invention are less than the limits set in the ICH guidelines. For example, the purified trehalose contains <5000 ppm ethanol (e.g., <140 ppm), and/or <3000 ppm methanol.

In a preferred embodiment, the Trehalose used in the combination product of the invention is anhydrous or hydrated, for example mono or dihydrated. In a most preferred embodiment, the Trehalose used in the combination product of the invention is Trehalose dihydrate.

Miglustat, on the other hand, is a well-known member of the family of N-alkylated imino sugars. It has been approved in several countries, including in EU and in the US (Butters et al, 2007). Synthesis of this molecule is explained for example in EP1896083. Only one polymorphic crystalline form was observed. This crystalline anhydrous form has been characterized by various analytical techniques like FTIR, XRPD (X-Ray Powder Diffraction), DSC (Differential Scanning Calorimeter) and TGA (Thermo Gravimetric Analysis) during the manufacture and at release.

The term "Miglustat" as used herein, therefore refers to the compound N-butyl-deoxynojirimycin (N-butyl DNJ), also named 1,5-(butylimino)-1,5-dideoxy-D-glucitol, N-butyl-deoxynojirimycin or (2R,3R,4R,5S)-1-butyl-2-(hydroxymethyl)piperidine-3,4,5-triol, as well as any other form such as a salt, polymorph, enantiomer, stereoisomer, conformer, solvate, ester, amide, prodrug, analog, derivative, or the like, provided said salt, enantiomer, stereoisomer, conformer, solvate ester, amide, prodrug, analog, or derivative is capable of inhibiting the Trehalase enzyme and/or act as a chaperone protein for Trehalose.

The characteristics of the two components of the combination product of the invention are more preferably those summarized on Table I:

| **Structural formula** | Trehalose dihydrate<br>Miglustat |
|---|---|
| **Chemical name** | Trehalose: α,α-trehalose dihydrate: α-D-glucOpyranosyl α-D-glucopyranoside dihydrate<br>Miglustat: (2R,3R,4R,5S)-1-butyl-2-(hydroxyniethyl) piperidine-3.4.5-triol |
| **Molecular formula** | Trehalose : $C_{12}H_{22}O_{11}•2H_2O$<br>Miglustat: $C_{10}H_{21}NO_4$ |
| **Molecular weight** | Trehalose: 378.33<br>Miglustat: 219.281 |
| **CAS Reference** | Trehalose: 6138-23-4<br>Miglustat: 72599-27-0 |

(continued)

| | |
|---|---|
| **Physiochemical description** | Trehalose: White, odorless, non-hygroscopic crystalline powder<br>Miglustat: White crystalline powder |
| **Solubility** | Trehalose: 68.9 g/100 g $H_2O$ at 20°C<br>Miglustat: Highly soluble in water (>1000 mg/mL as a free base). |

In the combination product of the invention, it is also possible to replace Miglustat by another inhibitor of the glucosylceramide synthase enzyme, such as Lucerastat and Venglustat (see details below).

The term "lysosomal storage disorders and disorders characterized by lysosomal dysfunction" may be used herein to describe any condition that may be caused by impaired lysosomal metabolism or any condition which exhibits or is exacerbated by lysosomal dysfunction. There are at least 60 known lysosomal storage disorders and many other disorders characterized by lysosomal dysfunction which may affect different parts of the body, including the skeleton, brain, skin, heart, and central nervous system.

Additional disorders characterized by lysosomal dysfunction continue to be identified. Some of them are highlighted on Table II below.

| Disease | Deficiency |
|---|---|
| ***Primary lysosomal hydrolase defect*** | |
| Gaucher disease | Glucocerebrosidase |
| GM1 gangliosidosis | GM1-β-galactosidase |
| Tay – Sachs disease | β-Hexosaminidase A |
| Sandhoff disease | β-Hexosaminidase A+B |
| Fabry disease | α-Galactosidase A |
| Krabbe disease | β-Galactosyl ceramidase |
| Niemann–Pick disease Types A and B | Sphingomyelinase |
| Metachromatic leukodystrophy | Arylsulphatase A |
| MPS IH (Hurler syndrome) | α-Iduronidase |
| MPS IS (Scheie syndrome) | α-Iduronidase |
| MPS II (Hunter syndrome) | Iduronate sulphatase |
| MPS IIIA (Sanfilippo A syndrome) | Heparan sulphamidase |
| MPS IIIB (Sanfilippo B syndrome) | N-Acetylglucosaminidase |
| MPS IIIC (Sanfilippo C syndrome) | Acetyl CoA:α-glucosaminide *N*-acetyltransferase |
| MPS IIID (Sanfilippo D syndrome) | *N*-acetyl glucosamine-6-sulphatase |
| MPS IV A (Morquio A disease) | Acetyl galactosamine-6-sulphatase |
| MPS IVB (Morquio B disease) | β-Galactosidase |
| MPS V (redesignated MPS IS) | |
| MPS VI (Maroteaux Lamy Syndrome) | Acetyl galactosamine-4-sulphatase (ARSB) |
| MPS VII (Sly Syndrome) | β-Glucuronidase |
| MPS IX | Hyaluronidase |
| Farber disease | Acid ceramidase |
| Cholesteryl ester storage disease | Acid lipase |
| Pompe disease (type II) | α1,4-glucosidase |
| Aspartylglucosaminuria | Glycosylasparaginase |
| Fucosidosis | α-Fucosidase |
| α-Mannosidosis | α-Mannosidase |
| β-Mannosidosis | β-Mannosidase |
| Schindler disease | *N*-acetylgalactosaminidase |
| Sialidosis | α-Neuraminidase |
| Infantile neuronal ceroid lipofuscinoses (CLN1) | Palmitoyl protein thioesterase |
| Late infantile neuronal ceroid lipofuscinosis (CLN2) | Carboxypeptidase |

| Disease | Deficiency |
|---|---|
| ***Post-translational processing defect in lysosomal enzymes*** | |
| Mucosulphatidosis (MSD) | Multiple sulphatases |
| ***Trafficking defect in lysosomal enzymes*** | |
| Mucolipidosis type II (I -cell disease) | *N*-acetyl glucosamine phosphoryl transferase |
| Mucolipidosis type IIIA (pseudo-Hurler polydystrophy) | *N*-acetyl glucosamine phosphoryl transferase |
| Mucolipidosis type IIIC | |
| ***Defect in lysosomal enzyme protection*** | |
| Galactosialidosis | Protective protein cathepsin A (PPCA) (β-galactosidase and neuraminidase) |
| ***Defect in soluble non-enzymatic lysosomal proteins*** | |
| Niemann–Pick type C | NPC2 |
| GM2 activator protein deficiency, Variant AB | GM2 activator protein |
| Sphingolipid activator protein (SAP) deficiency | Sphingolipid activator protein |
| Neuronal ceroid lipofuscinosis (CLN5) | |
| ***Transmembrane (non-enzyme) protein defect*** | |
| Danon disease | Lysosome-associated membrane protein 2 (LAMP2) |
| Niemann–Pick Type C | NPC1 |
| Cystinosis | Cystinosin |
| Infantile free sialic acid storage disease (ISSD) | Sialin |
| Salla disease (free sialic acid storage) | Sialin |
| Juvenile neuronal ceroid lipofuscinosis (CLN3, Batten disease) | |
| Neuronal ceroid lipofuscinoses (CLN6 and CLN8) | |
| Mucolipidosis type IV | Mucolipin |
| ***Unclassified*** | |
| Neuronal ceroid lipofuscinoses (CLN4 and CLN7) | |

Non-limiting examples of lysosomal storage disorders and disorders characterized by lysosomal dysfunction that may be treated using combination product and methods of the present disclosure include : Juvenile Neuronal Ceroid Lipofuscinosis (JNCL, juvenile Batten or CLN3 disease), Aspartylglucosaminuria, Cystinosis, Fabry Disease, San Filippo disease, Gaucher Disease Types I, II, and III, Glycogen Storage Disease II (Pompe Disease), GM2-Gangliosidosis Type I (Tay Sachs Disease), GM2-Gangliosidosis Type II (Sandhoff Disease), Metachromatic Leukodystrophy, Mucolipidosis Types I, II/III and IV, Mucopolysaccharide Storage Diseases, Niemann-Pick Disease Types A/B, C1 and C2, Schindler Disease Types I and II, CLN1 disease, CLN2 disease, CLN4 disease, CLN5 disease, CLN6 disease, CLN7 disease, CLN8 disease, CLN10 disease, CLN11 disease, CLN12 disease, CLN13 disease, and CLN14 disease.

The mucopolysaccharide storage disease is preferably selected from : Hurler syndrome (MPS IH), Hurler-Scheie syndrome (MPS IH/S), Scheie syndrome (MPS IS; Mucopolysaccharidosis type V), Hunter syndrome (MPS II), Sanfilippo syndrome A (MPS IIIA), Sanfilippo syndrome C (MPS IIIC), Sanfilippo syndrome D (MPS IIID), Morquio Type A, Morquio Type B, Maroteaux-Lamy (MPS VI), Sly diseases (MPS VII), and Natowicz syndrome (MPS IX).

In a preferred embodiment, the combination product of the invention enables to treat Juvenile Neuronal Ceroid Lipofuscinosis (JNCL, juvenile Batten or CLN3 disease), Aspartylglucosaminuria, Cystinosis, San Filippo disease, Glycogen Storage Disease II (Pompe Disease), Metachromatic Leukodystrophy, Mucolipidosis Types I, II/III and IV, Mucopolysaccharide Storage Diseases, Niemann-Pick Disease Types A/B, C1 and C2, Schindler Disease Types I and II, CLN1 disease, CLN2 disease, CLN4 disease, CLN5 disease, CLN6 disease, CLN7 disease, CLN8 disease, CLN10 disease, CLN12 disease, CLN13 disease, and CLN14 disease.

In a preferred embodiment, the combination product of the invention enables to treat Neuronal Ceroid Lipofuscinosis (CLN disease), in particular CLN1 disease, CLN2 disease, CLN3 disease, CLN4 disease, CLN5 disease, CLN6 disease, CLN7 disease, CLN8 disease, CLN10 disease, CLN11 disease, CLN12 disease, CLN13 disease, or CLN14 disease.

In a most preferred embodiment, the combination product of the invention enables to treat Juvenile Neuronal Ceroid Lipofuscinosis (JNCL, or Batten disease, or the CLN3 form of Batten disease). Although it shares some pattern with other CLN diseases, this disease is still very complicated to understand, due to the fact that the CLN3 primary function is still unknown, and because this protein has multiple interaction partners (Getty A.L. and Pearce D.A., 2011).

JNCL is the most prevalent neurodegenerative disorder of childhood. A hallmark of JNCL is the intralysosomal accumulation of ceroid lipopigments in most nerve cells and in various extra-cerebral tissues, indicating impairment of autophagy-lysosome pathways. JNCL presents with vision failure and hearing loss, and progresses to include seizures, motor dysfunction, and dementia. JNCL patients experience relentless physical and cognitive decline that leads to death by the third decade of life. As such, treating JNCL using the combination product of the present invention will prevent intralysosomal accumulation of ceroid lipopigments in nerve cells and in various cerebral and extra-cerebral tissues of a subject having JNCL, or will reduce or eliminate intralysosomal accumulation of the ceroid lipopigments. Methods of determining intralysosomal accumulation of ceroid lipopigments are known in the art and may be as described in the examples. Additionally, treating JNCL using the combination product of the invention will prevent, reverse, or arrest cognitive decline in a subject. Methods of determining cognitive decline resulting from JNCL in a subject are known in the art. For instance, treating JNCL using the combination product of the invention may prevent, reverse, or arrest vision failure. Treating JNCL using the combination product of the invention may also prevent, reverse, or arrest hearing loss. Treating JNCL using the combination product of the invention may also reduce the severity and/or intensity of seizures. Additionally, treating JNCL using the combination product of the invention may improve or prevent motor dysfunction. Treating JNCL using the combination product of the invention may also improve or prevent dementia.

Treating JNCL using the combination product of the invention closure may also extend the lifespan of a subject in need thereof. Using the combination product of the invention, the median life span of a subject having JNCL may be extended by about 1%, 5%, 10%, 15%, 20%, 30%, 40%, 50%, 60%, 70%, 80%, or about 90% or to the point where the disorder no longer is a factor in longevity of the subject. For instance, the combination product of the invention may extend the median lifespan of a subject with JNCL by about 60%, 65%, 70%, 75%, 80%, 85%, or about 90% or to the point where the disorder no longer is a factor in longevity of the subject. Alternatively, the combination product of the invention may extend the median lifespan of a subject with JNCL by about 20%, 25%, 30%, 35%, 40%, 45% or about 50%. The combination product of the invention may also extend the median lifespan of a subject with JNCL by about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, or about 25%.

The term "parenterally" as herein defined refers to a route of administration where the desired effect is systemic and the active agent (herein defined as Trehalose), is administered by routes other than the digestive tract, for example intravenous, subcutaneous, intradermal, intramuscular and intraperitoneal administration. In a preferred embodiment, the composition containing Trehalose is for intravenous or subcutaneous administration.

In the combination product of the invention, Trehalose is contained in a composition that is suitable for parenteral administration. Such compositions are known in the art. This composition contains, apart from Trehalose, at least one pharmaceutically acceptable additive, carrier, excipient or diluent.

In a preferred embodiment, Trehalose is the single active principle in the composition for parenteral administration that is contained in the combination product of the invention.

In a preferred embodiment, the composition of the invention comprising Trehalose is formulated as an injectable intravenous dosage form for intravenous administration. It can be, for example, formulated as a dry powder that has to be diluted extemporaneously in the appropriate pharmaceutically acceptable solution.

In a more preferred embodiment, the dosage form is an infusion fluid comprising Trehalose. Trehalose infusion fluid formulations are known in the art. A Trehalose infusion fluid can typically comprise from about 1 to about 1000 mg/mL Trehalose, from about 10 to about 500 mg/mL Trehalose, or from about 50 to about 150 mg/mL Trehalose. In some aspects, a Trehalose infusion fluid according to the invention can comprise from about 80 to about 100 mg/mL Trehalose. In some embodiments, the concentration of Trehalose in the formulation in the combination product of the invention is between about 0.1 % (w/v) to about 50 % (w/v), preferably between 5 % (w/v) to about 15 % (w/v), in particular of about 8-10 % (w/v). In some embodiments, the injectable composition comprising Trehalose has an osmolality of from about 280 to

about 330 mOsm/Kg.

When administered intravenously, the Trehalose can be administered in said subject at a dosage ranging from about 0.25 g/kg to about 1 g/kg, preferably from about 0.25 g/kg to about 0,75 g/kg. At such doses, Trehalose is preferably administered once or twice weekly, more preferably once weekly. In other embodiments, an intravenous Trehalose composition is administered at a dose ranging from about 0.1 g/kg to about 1 g/Kg or from about 0.2 g/kg to about 0.8 g/Kg.

When administered intravenously, a composition comprising Trehalose may be administered over a period of about 25, 50, 60, 70, 75, 80, 85, 90, 95 to about 120 minutes or 180 minutes (three hours). More preferably, when administered intravenously, a composition comprising Trehalose may be administered within less than 90 minutes, preferably for about 25 minutes to about three hours, more preferably for about 40 minutes to about 90 minutes, most preferably for about 50 minutes to about 70 minutes.

The injectable composition of the invention, comprising Trehalose or a Trehalose analog, preferably comprises a low level of endotoxins. Bacterial endotoxins are lipopolysaccharides (LPS), components of Gram-negative bacterial cell walls known to cause fevers and disease when injected into the bloodstream. Bacterial endotoxins are heat stable and toxicity is not dependent on the presence of the bacterial cell. Since many therapeutics, including Trehalose, may be made in bacteria, endotoxin testing is employed to ensure a therapeutic product is endotoxin-free. A composition comprising Trehalose may contain less than 1.0, 0.9, 0.8, 0.75, 0.7, 0.6, 0.5, 0.4, 0.3, 0.2, 0.1 or less endotoxin units per mL. Preferably, a composition comprising Trehalose contains less than 0.75 endotoxin units per mL of solution.

Further, a composition comprising Trehalose is preferably administered intravenously so that the maximum endotoxin level is less than 5 EU per kilogram of body weight per hour. In particular, a composition comprising Trehalose may be administered intravenously such that the endotoxin level is less than about 1, 2, 3, or less than about 4 endotoxin units per kilogram of body weight per hour.

In one particular embodiment, the Trehalose of the combination product of the invention is administered once weekly by an intravenous infusion extending for about 50 minutes to about 70 minutes. In one most preferred embodiment, the Trehalose is administered once weekly at a dosage ranging from about 0.25 g/Kg to about 0.75 g/kg, by an intravenous infusion extending for about 50 to about 70 minutes.

On another hand, in the context of the invention, the Miglustat can be administered to the subjects in need thereof by any oral formulations, such as in food, beverage, capsules, tablets, syrup, etc. Oral compositions generally may include an inert diluent or an edible carrier. Oral compositions may be enclosed in gelatin capsules or compressed into tablets. For the purpose of oral therapeutic administration, the active compound may be incorporated with excipients and used in the form of tablets, troches, or capsules. Oral compositions may also be prepared using a fluid carrier for use as a mouthwash, wherein the compound in the fluid carrier is applied orally and swished and expectorated or swallowed. Pharmaceutically compatible binding agents and/or adjuvant materials may be included as part of the composition. The tablets, pills, capsules, troches, and the like, may contain any of the following ingredients, or compounds of a similar nature: a binder such as microcrystalline cellulose, gum tragacanth or gelatin; an excipient such as starch or lactose; a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. For administration by inhalation, the compounds are delivered in the form of an aerosol spray from a pressured container or dispenser which contains a suitable propellant, e.g., a gas such as carbon dioxide, or a nebulizer.

Preferably, in the combination product of the invention, Miglustat is administered in a capsule. Capsules of Miglustat are commercialized by Actelion Pharmaceuticals under the name Zavesca®. The nonclinical and clinical safety of this product in patients is well-established. In addition, Zavesca is approved in the EU for safe use in adults and children with Niemann-Pick Type C disease, a related neurodegenerative lysosomal storage disease at doses up to 200 mg TID.

In the combination product of the invention, Miglustat can be administered at a dosage ranging from about 75 mg per day to about 600 mg per day. In a preferred embodiment, Miglustat is administered at a dosage ranging from about 75mg per day to 600 mg per day.

When Trehalose is administered in combination with Miglustat, the oral dosage form for Miglustat is preferably one or more capsule(s), each comprising from about 10 to about 500 mg Miglustat, or from about 50 to about 300 mg Miglustat. In some aspects, a Miglustat capsule that can be used in the combination product of the invention can comprise 100, 150, 200, 250 or 300mg of Miglustat.

In an even more preferred embodiment, Miglustat in the combination product of the invention is administered three times per day (t.i.d.) to six times per day with three to six capsules containing each between 90 and 110 mg of Miglustat, preferably 100 mg of Miglustat.

More precisely, the Miglustat of the combination product of the invention can be orally administered :

- at a dosage ranging from about 175 mg once a day to about 300 mg t.i.d if the subject is twelve years of age or older or if the subject is less than twelve years of age and has a body surface area (BSA) of > 1.25 $m^2$,
- at a dosage ranging from about 175 mg once a day to about 300 mg twice a day if the subject is less than twelve years of

age and has a BSA of > 0.88-1.25 $m^2$,

- at a dosage ranging from about 75 mg once a day to about 150 mg t.i.d. if the subject is less than twelve years of age and has a BSA of > 0.73-0.88 $m^2$,
- at a dosage ranging from about 75 mg once a day to about 150 mg twice a day if the subject is less than twelve years of age and has a BSA of > 0.47-0.73 $m^2$,
- or at a dosage ranging from about 75 mg to about 150 mg once a day if the subject is less than twelve years of age and has a BSA of > 0.47 $m^2$.

In some specific embodiments of the combination product of the invention, Trehalose is intravenously administered once weekly at a dosage ranging from about 0.25 g/Kg to about 0.75 g/Kg by an intravenous infusion extending for about 50 to about 70 minutes and the Miglustat is orally administered at a dosage ranging from about 100 mg t.i.d to about 100 mg six times per day, or at any lower dosage described above, depending on the age, and body surface area of the subject in need of the treatment.

In a particular embodiment, the present disclosure provides a method of treating juvenile Neuronal Ceroid Lipofuscinosis (Batten Disease or CLN3) in a subject in need thereof, the method comprising administering Trehalose intravenously once weekly at a dosage ranging from 0.25 g/Kg to about 0.75 g/Kg by an intravenous infusion extending for about 50 to about 70 minutes and orally administering Miglustat at a dosage ranging from about 175 mg once a day to about 300 mg t.i.d if the subject is twelve years of age or older or if the subject less than twelve years of age and has a body surface area (BSA) of > 1.25 $m^2$, at a dosage ranging from about 175 mg once a day to about 300 mg twice a day if the subject is less than twelve years of age and has a BSA of > 0.88-1.25 $m^2$, at a dosage ranging from about 75 mg once a day to about 150 mg t.i.d. if the subject is less than twelve years of age and has a BSA of > 0.73-0.88 $m^2$, at a dosage ranging from about 75 mg once a day to about 150 mg twice a day if the subject is less than twelve years of age and has a BSA of > 0.47-0.73 $m^2$, or at a dosage ranging from about 75 mg to about 150 mg once a day if the subject is less than twelve years of age and has a BSA of > 0.47 $m^2$.

Treating JNCL with inhibitors of the glycosphingolipid synthesis

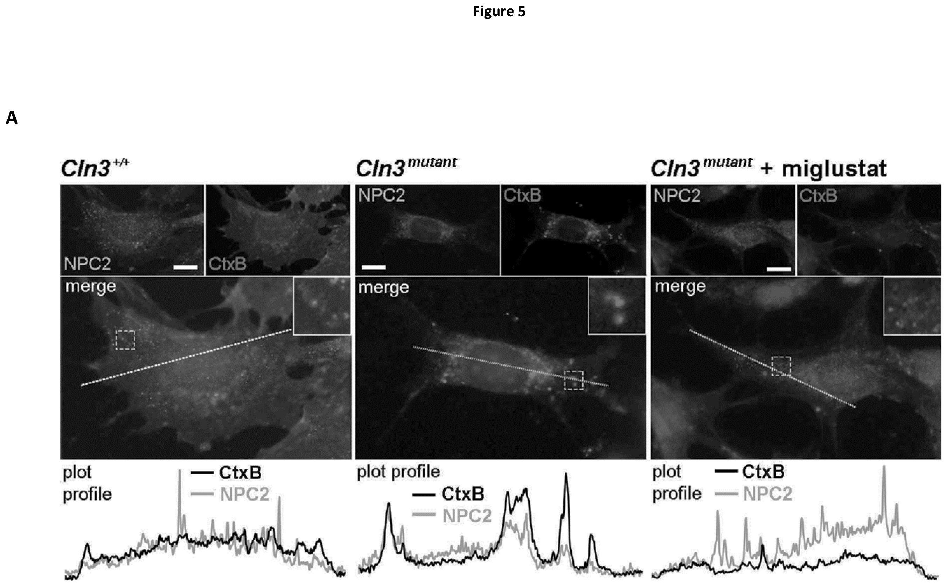

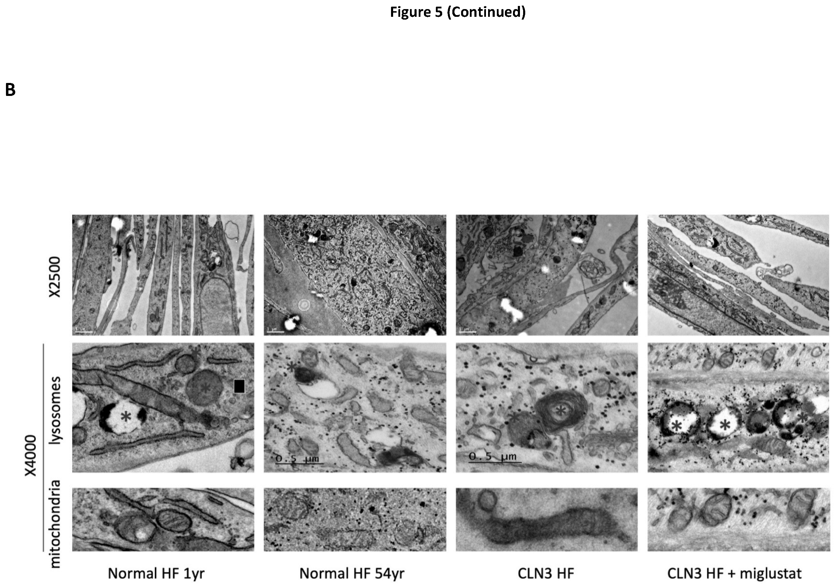

As disclosed previously, the present inventors have shown that, surprisingly, Miglustat has a strong effect, when administered alone, on neuronal cell death, neuroinflammation and microglial activation, even at low dose (Figures 1-3). As shown in example 5 and figures 5-7, this effect could be linked to the regulatory activity of Miglustat on the glycosphingolipid synthesis, more precisely on its ability to reduce abnormal ganglioside accumulation in lysosomes from CLN3 deficient cells.

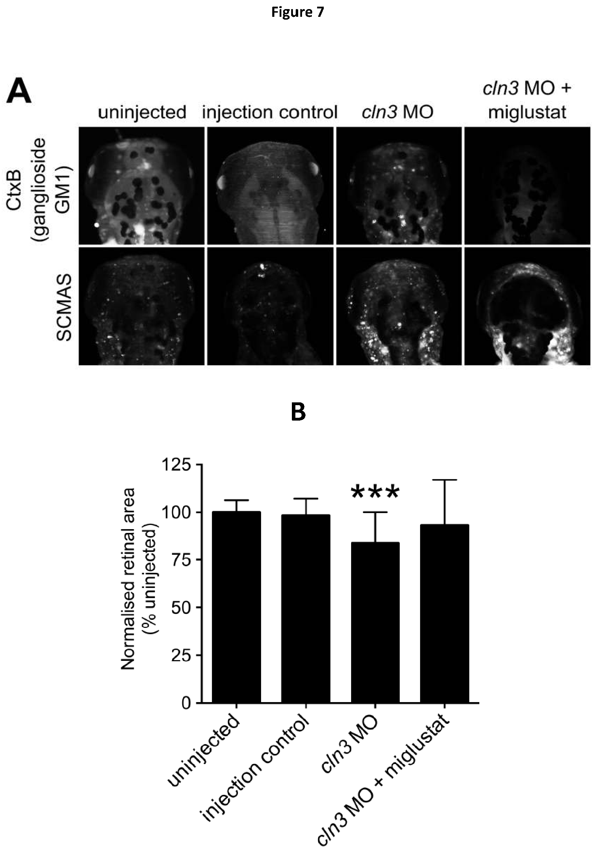

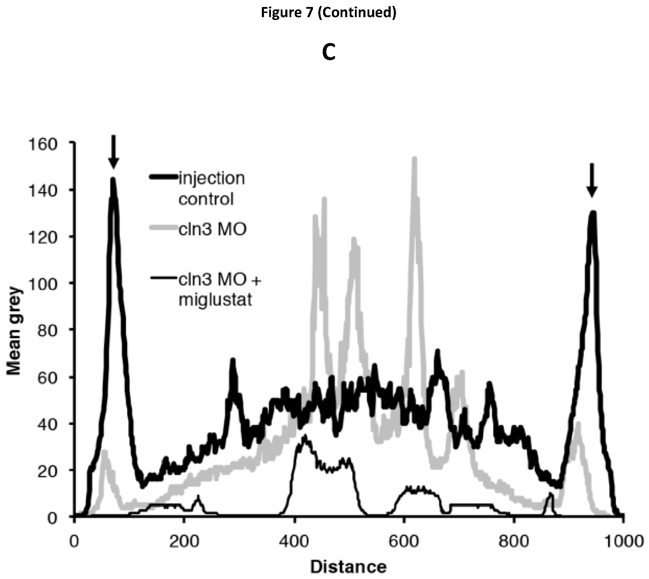

In particular, the present data (example 5) show for the first time that miglustat not only reduces GSL storage but in doing so also normalises organelle health, function, cellular signalling and restores normal SCMAS protein processing within the lysosome of human CLN3 deficient cells. Also, the present data show that administration of miglustat can reverse the impact of CLN3 mutation on reported zebrafish developmental phenotypes, including retinal area (Figure 7). Visual abnormalities are common in the NCLs so this is potentially a very important disease modifying phenotype as it illustrates normalisation of a key component of CLN3 pathophysiology.

In a second aspect, the present inventors thus propose to administer Miglustat or any other glucosylceramide synthase inhibitor, as single active principle, for reducing ganglioside accumulation and inflammation in neuronal cells patients suffering from the Juvenile Neuronal Ceroid Lipofuscinosis (JNCL disease). More precisely, said inhibitor can be used for reducing neuronal cell death, neuroinflammation, microglial activation and lysosomal glycosphingolipid storage in the cerebellum of patients suffering from the Juvenile Neuronal Ceroid Lipofuscinosis (JNCL disease). It can be used more generally to restore normal function in these cells.

This particular regimen is likely to display the same therapeutic efficacy in the treatment of disorders involving abnormal lysosomal storage, and the CLN3 form of the Batten disease in particular, as the combination product of the invention disclosed above.

In this second aspect, the present invention therefore relates to a glucosylceramide synthase inhibitor such as Miglustat for its use for treating the JNCL disease in a subject in need thereof (see above for detailed description of this disease). In a preferred embodiment, said glucosylceramide synthase inhibitor is administered orally.

The present invention also discloses a method for treating or alleviating the JNCL disease in a human subject in need thereof, said method comprising administering to said subject a therapeutically effective amount of a glucosylceramide synthase inhibitor or of a pharmaceutical formulation comprising a therapeutically effective amount of said inhibitor.

The glucosylceramide synthase enzyme (also known as UDP-glucose:ceramide glucosyltransferase, or EC 2.4.1.80) is an enzyme inherent to glycosphingolipid metabolism, that catalyzes the transfer of glucose to ceramide, the first committed step in glycolipid biosynthesis (Platt et al, JBC 1994). Any inhibitor of this enzyme can be used in the context of the present invention, in particular, Miglustat, Lucerastat, Venglustat, Eliglustat, GZ667161 (a Venglustat analog also called Ibiglustat), ACT-519276 (a Lucerastat analog, also called Sinbaglustat), AMP-DNM (adamantane-pentyl-deox-

ynojirimycin), AZ-3102, analogs or derivatives thereof.

As disclosed previously, the term "Miglustat" refers to the compound N-butyl-deoxynojirimycin (N-butyl DNJ), also named 1,5-(butylimino)-1,5-dideoxy-D-glucitol, N-butyl-deoxynojirimycin or (2R,3R,4R,5S)-1-butyl-2-(hydroxymethyl)piperidine-3,4,5-triol, as well as any other form such as a salt, polymorph, enantiomer, stereoisomer, conformer, solvate, ester, amide, prodrug, analog, derivative, or the like, provided said salt, enantiomer, stereoisomer, conformer, solvate ester, amide, prodrug, analog, or derivative is capable of inhibiting the glucosylceramide synthase enzyme efficiently.

The term "Lucerastat" herein refers to the compound N-butyl deoxygalactojirimycin (NB DGJ), also named ACT-434964, OGT-923, and CDP-923, as well as any other form such as a salt, polymorph, enantiomer, stereoisomer, conformer, solvate, ester, amide, prodrug, analog, derivative, or the like, provided said salt, enantiomer, stereoisomer, conformer, solvate ester, amide, prodrug, analog, or derivative is capable of inhibiting the glucosylceramide synthase enzyme efficiently. Said analog can be, in particular, ACT-519276 (also called Sinbaglustat).

The term "Venglustat" herein refers to the compound GZ-402671, SAR-402671, and ibiglustat,, as well as any other form such as a salt, polymorph, enantiomer, stereoisomer, conformer, solvate, ester, amide, prodrug, analog, derivative, or the like, provided said salt, enantiomer, stereoisomer, conformer, solvate ester, amide, prodrug, analog, or derivative is capable of inhibiting the glucosylceramide synthase enzyme efficiently. Said analog can be, in particular, GZ667161 (also called Ibiglustat).

The present invention also targets a pharmaceutical composition containing any of said inhibitor of the glucosylceramide synthase enzyme, and a pharmaceutically acceptable excipient, for use for treating in particular the JNCL disease (or Batten disease, or CLN3 disease).

Treating JNCL using this pharmaceutical composition may extend the lifespan of a subject in need thereof by about 1%, 5%, 10%, 15%, 20%, 30%, 40%, 50%, 60%, 70%, 80%, or about 90% or to the point where the disorder no longer is a factor in longevity of the subject. Alternatively, this pharmaceutical composition may extend the median lifespan of a subject with JNCL by about 20%, 25%, 30%, 35%, 40%, 45% or about 50%.

In this pharmaceutical composition, the inhibitor of the glucosylceramide synthase enzyme such as Miglustat, Lucerastat, Venglustat, etc. is preferably contained in a composition that is suitable for oral administration. Such compositions are known in the art. This composition contains, apart from the inhibitor, at least one pharmaceutically acceptable additive, carrier, excipient or diluent.

In one embodiment, Miglustat (and/or the other inhibitor of the glucosylceramide synthase enzyme) is /are the single active principle in the pharmaceutical composition of the invention. In other terms, the composition of the invention contains, in this embodiment, the inhibitor(s) of glucosylceramide synthase enzyme but no other active principle. In a preferred embodiment, the composition of the invention contains several inhibitors of the glucosylceramide synthase enzyme. In a more preferred embodiment, the composition of the invention contains only one inhibitor of glucosylceramide synthase enzyme, for example Miglustat.

As shown in the examples below, the effect of Miglustat on neuronal cell death is strong, and this strong effect is even more pronounced in the presence of Trehalose.

Therefore, in another embodiment, the inhibitor of the glucosylceramide synthase enzyme is administered together with Trehalose, as disclosed above. The two administrations are performed separately, preferably via two different routes of administration (Trehalose being preferably administered parenterally, as described above). The two administrations can be done simultaneously or in a staggered manner.

In this aspect of the invention, Miglustat can be administered to the subjects in need thereof by any oral formulations, such as in food, beverage, capsules, tablets, syrup, etc. Gastro-resistant tablets containing e.g., functional polymers, are herein preferred.

Oral compositions may also be prepared using a fluid carrier for use as syrup or a mouthwash, wherein the compound in the fluid carrier is applied orally and swished and expectorated or swallowed. Pharmaceutically compatible binding agents and/or adjuvant materials may be included as part of the composition.

The tablets, pills, capsules, troches, and the like, may contain any of the following ingredients, or compounds of a similar nature: a binder such as microcrystalline cellulose, gum tragacanth or gelatin; an excipient such as starch or lactose; a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

Miglustat is more preferably administered in a capsule. Capsules of Miglustat are commercialized by Actelion Pharmaceuticals under the name Zavesca®. The nonclinical and clinical safety of this product in patients is well-established. In addition, Zavesca is approved in the EU for safe use in adults and children with Niemann-Pick Type C disease, a related neurodegenerative lysosomal storage disease at doses up to 200 mg TID.

In this aspect of the invention, Miglustat can be administered at a dosage ranging from about 20mg per day to about 600mg per day, preferably from about 50mg per day to about 600 mg per day, more preferably from about 75 mg per day to about 600 mg per day and even more preferably from about 100mg per day to about 600mg per day.

In a most preferred embodiment, Miglustat is administered to the JNCL patient at a dosage ranging from about 100 mg per day to 600 mg per day, for example three times per day (t.i.d.) to six times per day with three to six capsules containing each between 90 and 110 mg of Miglustat, preferably 100 mg of Miglustat.

More precisely, the Miglustat can be orally administered :

- at a dosage ranging from about 175 mg once a day to about 300 mg t.i.d if the subject is twelve years of age or older or if the subject is less than twelve years of age and has a body surface area (BSA) of > 1.25 $m^2$,
- at a dosage ranging from about 175 mg once a day to about 300 mg twice a day if the subject is less than twelve years of age and has a BSA of > 0.88-1.25 $m^2$,
- at a dosage ranging from about 75 mg once a day to about 150 mg t.i.d. if the subject is less than twelve years of age and has a BSA of > 0.73-0.88 $m^2$,
- at a dosage ranging from about 75 mg once a day to about 150 mg twice a day if the subject is less than twelve years of age and has a BSA of > 0.47-0.73 $m^2$,
- or at a dosage ranging from about 75 mg to about 150 mg once a day if the subject is less than twelve years of age and has a BSA of > 0.47 $m^2$.

Definitions

As used herein, the term "treat" may be used to describe prophylaxis, amelioration, prevention or cure of a lysosomal storage disorder and disorders characterized by lysosomal dysfunction and/or one or more of its associated symptoms. For instance, treatment of an existing lysosomal storage disorder and disorders characterized by lysosomal dysfunction may reduce, ameliorate or altogether eliminate the disorder, or prevent it from worsening. Prophylactic treatment may reduce the risk of developing a disorder and/or lessen its severity if the disorder later develops.

A subject may be a rodent, a human, a livestock animal, a companion animal, or a zoological animal. In one embodiment, a subject may be a rodent, e.g., a mouse, a rat, a guinea pig, etc. In another embodiment, a subject may be a livestock animal. Non-limiting examples of suitable livestock animals may include pigs, cows, horses, goats, sheep, llamas and alpacas. In still another embodiment, a subject may be a companion animal. Non-limiting examples of companion animals may include pets such as dogs, cats, rabbits, and birds. In yet another embodiment, a subject may be a zoological animal. As used herein, a "zoological animal" refers to an animal that may be found in a zoo. Such animals may include non-human primates, large cats, wolves, and bears. In some preferred embodiments, a subject is a mouse. In other preferred embodiments, a subject is a human.

For the purpose of the invention, the term "pharmaceutically acceptable" is intended to mean what is useful to the preparation of a pharmaceutical composition, and what is generally safe and non-toxic, for a pharmaceutical use.

A "pharmaceutically acceptable carrier" or "excipient" or "solution" refers to a non-toxic solid, semi-solid or liquid filler, diluent, encapsulating material or formulation auxiliary of any type. In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Preferably, the pharmaceutical compositions of the invention contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The term "pharmaceutically acceptable salt" is intended to mean, in the framework of the present invention, a salt of a compound which is pharmaceutically acceptable, as defined above, and which possesses the pharmacological activity of the corresponding compound. The pharmaceutically acceptable salts comprise:

(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and (2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

Within the meaning of this invention, "stereoisomers" is intended to designate diastereoisomers or enantiomers. These are therefore optical isomers. Stereoisomers which are not mirror images of one another are thus designated as "diastereoisomers," and stereoisomers which are non-superimposable mirror images are designated as "enantiomers".

Within the meaning of this invention, "conformers" is intended to designate a form of stereoisomers in which the isomers can be interconverted just by rotations about formally single bonds.

## FIGURE LEGENDS

**Figure 1** shows that the apoptosis is reduced in multiple regions of the brain of treated Cln3$^{-/-}$ mice, as shown by quantification of the number of nuclei reactive to cleaved caspase 3 antibody per area analyzed : in thalamus VPM/VPL (A), in thalamus dLG (B), in the Cortex S1BF (C) and in the Cortex V1 (D). The statistical significance was determined by a linear mixed effects statistical model: *p < 0.05, **p < 0.01, ***p < 0.001. (E) shows the effect of orally administered Trehalose, Miglustat, alone, and a combination of Trehalose and Miglustat, on neuronal cell death in the VPM / VPL area of CLN3$^{-/-}$ mice. Four male mice / group were analyzed. Data are presented as mean ± standard deviation. H= 7.2g high dose of Miglustat / kg chow; KO = knock-out; L= low dose of 1,2g Miglustat / kg chow; VPL = ventral posterolateral nucleus; VPM = ventral posteromedial nucleus.

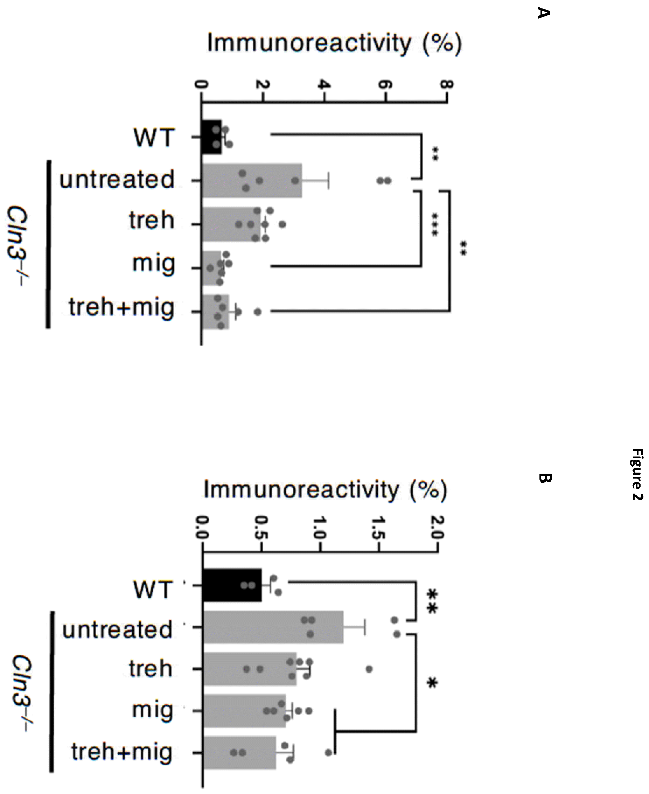

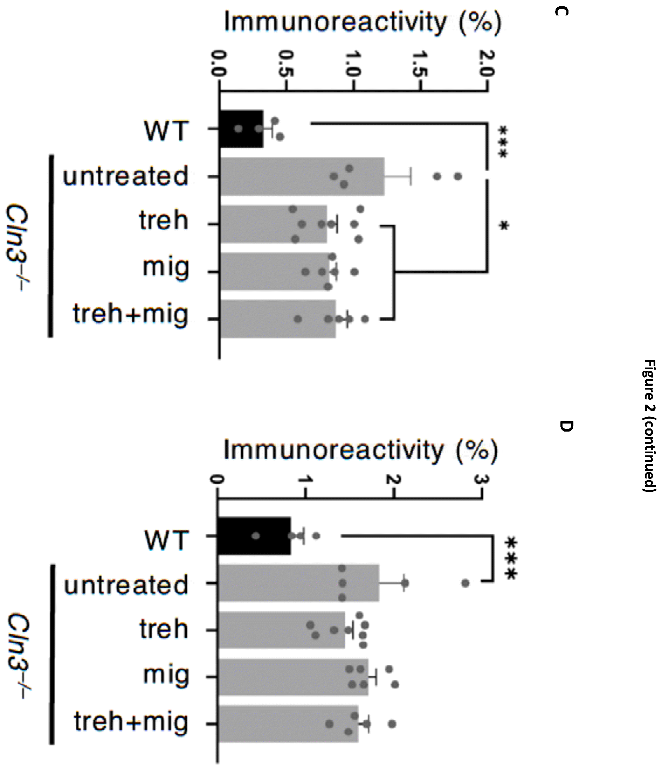

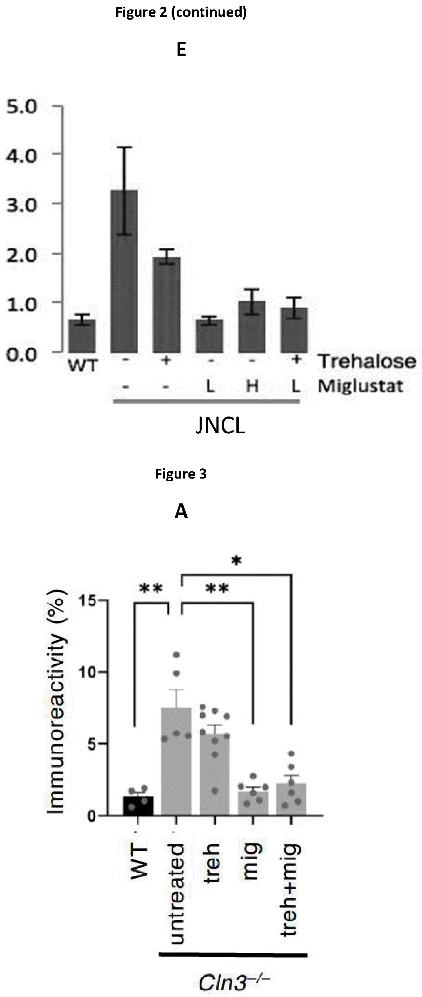

**Figure 2** shows the effect of Trehalose, Miglustat alone, and a combination of Trehalose and Miglustat on the microglial activation (CD68+ reactivity) within the thalamic ventral posterior medial and lateral nuclei (VPM/VPL) in Batten mice at 12 months of age (A) and dLG (B) regions of the thalamus and in S1BF (C) and V1(D) regions of the cortex of test and control mice. (E) shows the result with Low (L) or High (H) level of Miglustat on the VPM/VPL region of the brain (H= 7.2g high dose of Miglustat / kg chow; L= low dose of 1,2g Miglustat / kg chow). Four male mice / group were analyzed. Data are presented as mean ± standard deviation. Upon these treatments, Microglial activation levels are reduced in multiple regions of the brain of treated Cln3$^{-/-}$ mice, as shown by quantification of the percentage of area reactive to CD68 antibody. The statistical significance was determined by two-way ANOVA: *p < 0.05, **p < 0.01, ***p < 0.001.

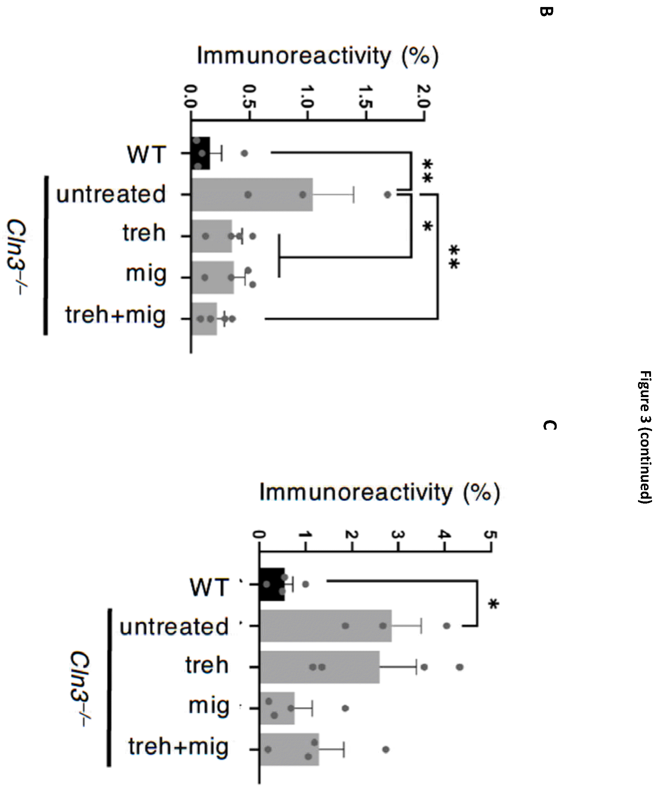

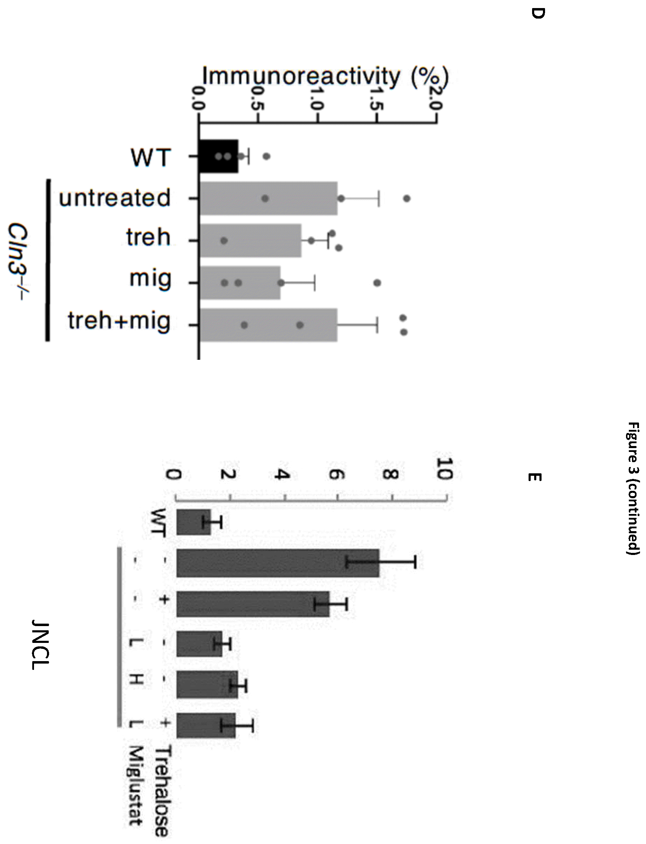

**Figure 3** shows the astrocyte activation levels are reduced in multiple regions of the brain of treated Cln3$^{-/-}$ mice, as shown by quantification of the percentage of area that is reactive to GFAP antibody in the VPM/VPL (A) and dLG (B) regions of the thalamus, and S1BF (C) and V1 (D) of the cortex of test and control mice. Representative images for each region and treatment are reported. The statistical significance was determined by two-way ANOVA: *p < 0.05, **p < 0.01. The astroglial activation (GFAP staining) in the VPM/VPL region of untreated *Cln3*$^{\Delta ex7\text{-}8}$ mice at 12 months of age, which is partially prevented by Trehalose administration and largely suppressed by Miglustat alone and Miglustat/Trehalose administration. (E) shows the result with Low (L) or High (H) level of Miglustat on the VPM/VPL region of the brain (H= 7.2g high dose of Miglustat / kg chow; L= low dose of 1,2g Miglustat / kg chow).

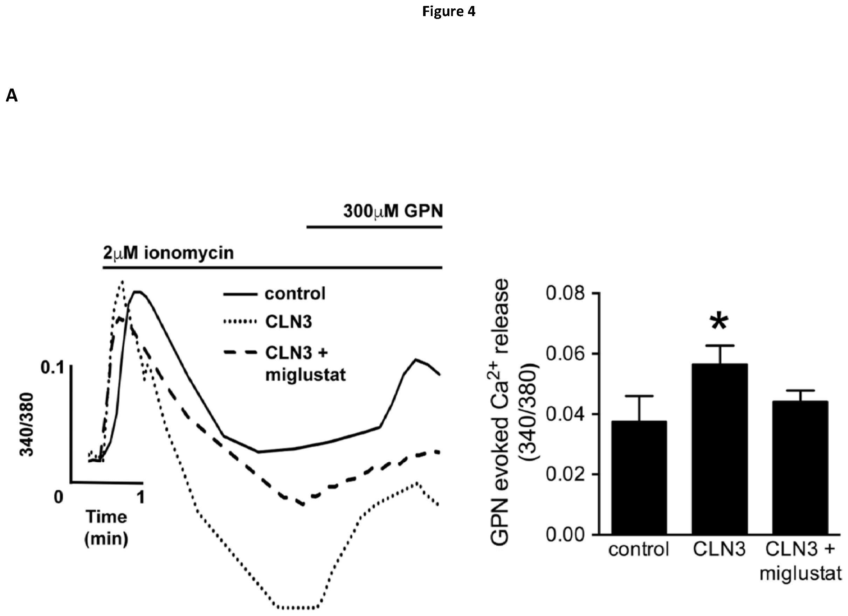

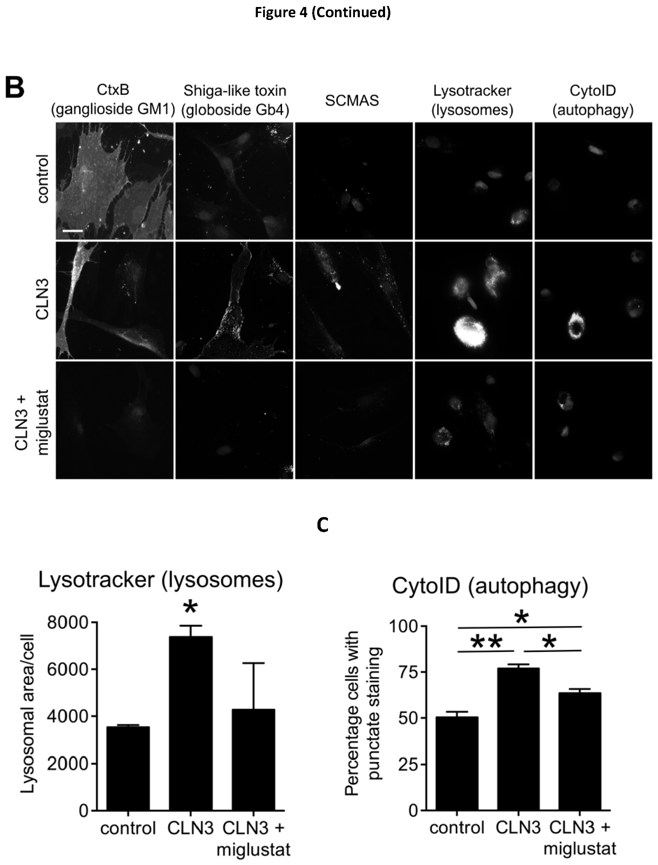

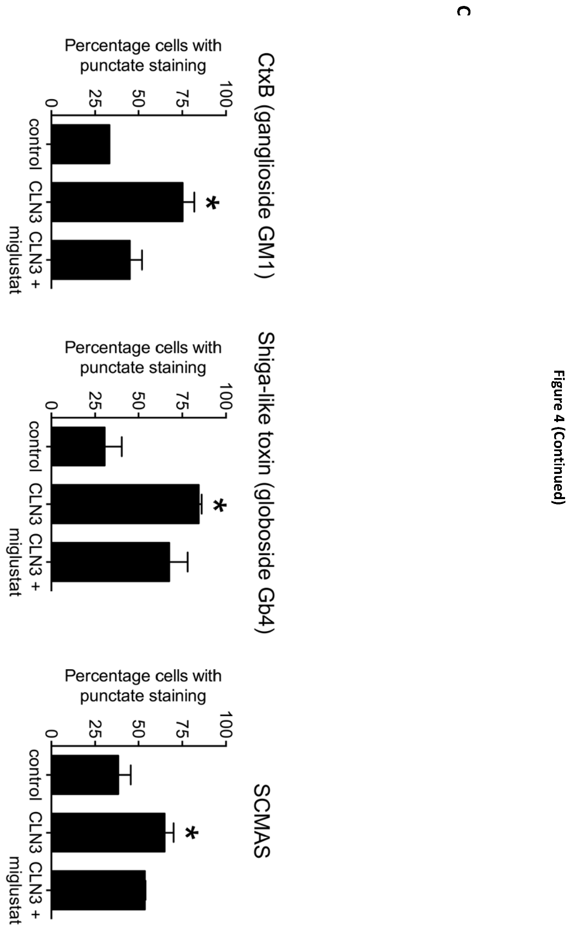

**Figure 4** shows that the glycosphingolipid storage is normalised by miglustat in CLN3 disease fibroblasts. (A) Lysosomal $Ca^{2+}$ content, measured using ionomycin to clamp non-lysosomal Ca2+ stores and GPN to burst lysosomes and allow cytosolic measurement of released Ca2+ using Fura 2,AM. (B) Representative images of 1kb deletion CLN3 fibroblasts (CRISPR generated) and age and passage matched controls (GM05399) that were fixed and stained for i) ganglioside GM1 using FITC-cholera toxin B subunit (CtxB), ii) globosides Gb3/Gb4 using Shiga-like toxin (SLxT), iii) subunit C of the mitochondrial ATP synthase (SCMAS) using anti-SCMAS antibodies, iv)

lysosomal expansion measured in live cells using lysotracker red and v) autophagy measured in live cells using CytoID. Nuclei were counterstained with Hoechst. All images were taken at the same exposure times and apart from the magnified areas were edited for brightness/contrast equally. Scale bar = 10μm. (C) Quantification (using ImageJ) of the mean grey area corresponding to the intensity of lysotracker red per cell and percentage of cells presenting with punctate cytoID, CtxB and SLTx across all the cells imaged. For all experiments, N=3 with an average of 70 cells imaged per repeat

**Figure 5** confirms the glycosphingolipid storage in CLN3 disease patient fibroblasts by confocal microscopy and TEM, and demonstrates normalisation following miglustat treatment. Representative images of CLN3 mutant fibroblasts and age and passage matched controls (GM05399, 1 year (1yr) old)) as well as a non-age matched control (54 year old (54yr)) sample that were fixed and processed for confocal imaging or TEM (A-B). (A) Confocal images of CtxB bound to ganglioside GM1 with the lysosomal marker anti-NPC2, below are line scans showing degree of overlap (co-localisation) between the two probes and from the indicated area in the above images. (B) TEM images of the indicated cell types with zooms of representative images of lysosomal and mitochondrial morphology. For (B), below are enlargements of areas within the indicated cell type. For all experiments, N=3 with an average of 25 cells imaged per repeat.

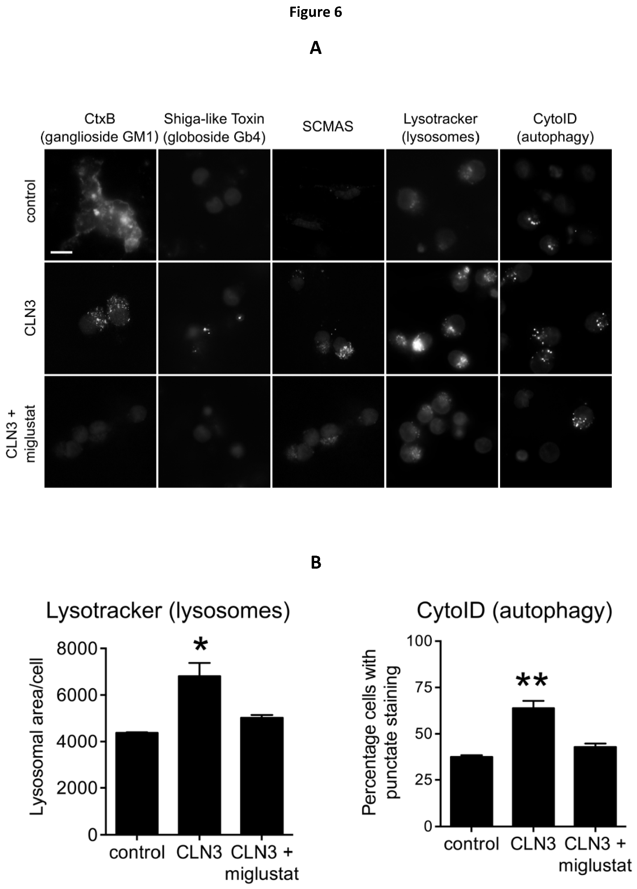

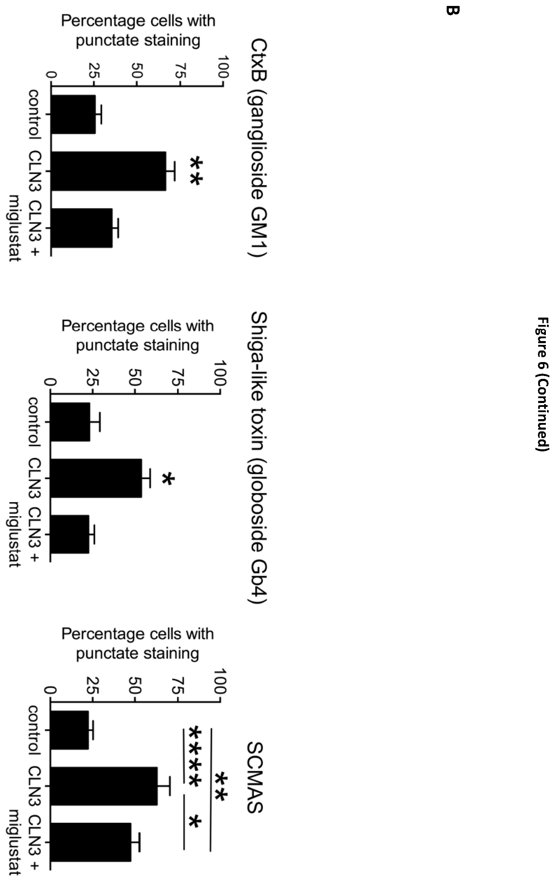

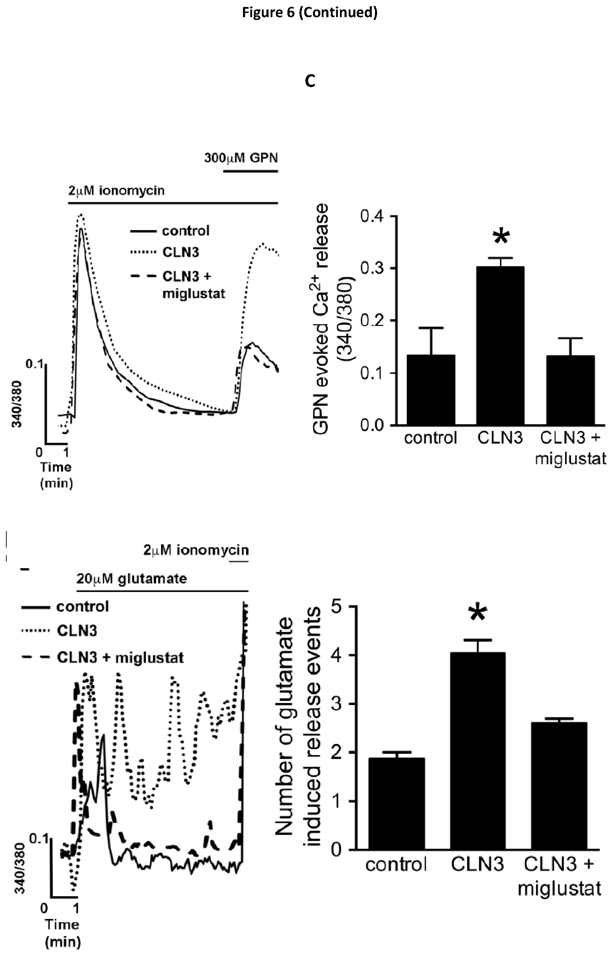

**Figure 6** shows the effect of miglustat on the Glycosphingolipid and lysosomal storage associated with CLN3 disease is normalised by miglustat in CLN3 patient iPSC derived cortical neurons. (A) Representative images of control and 1kb deletion CLN3 patient iPSC cells differentiated into neuronal progenitor cells and then over 4 further weeks into cortical neurons that were either fixed and stained for ganglioside GM1 using FITC-cholera toxin B subunit (CtxB), globoside Gb3 using Shiga-like toxin or SCMAS with anti-SCMAS antibody, or loaded live with lysotracker green or CytoID for 10min at 37°C followed by widefield fluorescence microscopy imaging. Nuclei were counterstained with Hoechst. Scale bars = 10μm. (B) Quantification (using ImageJ) of the mean grey area corresponding to the intensity of lysotracker green across all the cells imaged or the total number of cells with visible punctate staining counted. (C) Lysosomal $Ca^{2+}$ content, measured using ionomycin to clamp non-lysosomal Ca2+ stores and GPN to burst lysosomes and allow cytosolic measurement of released Ca2+ using Fura 2,AM and glutamate mediated excito-toxicity whereby cytosolic Ca2+ release is triggered using 20μM glutamate with subsequent spontaneous Ca2+ release events and return to baseline, or not in CLN3 (evidence of excitotoxicity) are recorded over time in the presence or absence of miglustat. For all experiments, N=3 with an average of 70 cells analysed per repeat.

**Figure 7** shows that the glycosphingolipid and lysosomal storage of lipofuscin associated SCMAS in cln3 disease morphant zebrafish is normalised by miglustat treatment. (A) Representative images of untreated control ($cln3^{+/+}$) tail-long (TL) zebrafish embryos or cln3 morphant (cln3morph) embryos injected with cln3 antisense morpholinos from the 2-4 cell stage (method of Wager et al, PLOS One, 2014) treated with or without miglustat (300μM) from 4 hours post fertilisation (hpf) until 92hpf. Fish were fixed and stained for either ganglioside GM1 using FITC-cholera toxin B subunit (CtxB) or antibodies against lipofuscin associated subunit C of the mitochondrial ATPase (SCMAS) and imaged by light sheet microscopy. (B) Retinal area measured using Zeiss Zen software from lightsheet images of the zebrafish embryos (different plane to (A)). N=3 with an average of 5 fish analysed per repeat. C, line scan of CtxB staining across the brain (from eye to eye) indicating high content of glycosphingolipids in the eyes in cln3+/+ fish (black arrows) and low content across the brain, cln3morphant fish have lower ganglioside GM1 in the eyes but have peaks across the brain indicative of storage which are reduced with miglustat treatment.

## EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### 1. MATERIAL AND METHODS

*Mouse colony and treatments*

$Cln3^{\Delta ex7\text{-}8}$ mice were obtained from the Jackson Laboratory. Control (C57BL/6J) and $Cln3^{\Delta ex7\text{-}8}$ mice were housed 3-4 per cage in a room with a 12-h light/12-h dark cycle. Food and water were provided ad libitum. All mice used in this study were analyzed at 8 and 12 months of age and were littermates produced by crossing heterozygous $Cln3^{\Delta ex7\text{-}8}$

mice. Only males were used for this analysis. Investigators were blinded when analyzing the data, and no randomization was necessary.

For oral administration, Trehalose was dissolved in drinking water to a final concentration of 2% and changed twice a week. Trehalose-containing water was given to *Cln3*$^{\Delta ex7\text{-}8}$ mice and WT mice by spontaneous oral administration starting at 21 days of age and continuing until the day the mice were sacrificed for neuropathology studies.

Miglustat was administered in mixture with the food pellet starting at 21 days of age and continuing until the day the mice were sacrificed for neuropathology studies. Miglustat food was prepared by TestDiet using the 5V5R rodent diet (LabDiet). Two concentrations were used: 1.2 g/Kg chow (indicated as "low concentration") and 7.2 g/Kg chow (indicated as "high concentration") and two separate groups of *Cln3*$^{\Delta ex7\text{-}8}$ mice were fed with either diet. One additional group of *Cln3*$^{\Delta ex7\text{-}8}$ mice was fed with a combination of the low-concentration Miglustat food and 2% Trehalose water.

*Immunohistochemistry*

Twelve-month-old homozygous *Cln3*$^{\Delta ex7\text{-}8}$ mice and age-matched controls (WT mice) were anaesthetized with isoflurane and transcardially perfused with PBS followed by 4% buffered paraformaldehyde in 0.1 M sodium phosphate buffer, pH 7.4. Brains were subsequently removed and postfixed overnight. Before sectioning, the brains were cryoprotected in a solution containing 30% sucrose in Tris-buffered saline (TBS: 50 mM Tris, pH 7.6). Consecutive 40 μm floating coronal sections were collected in 96-well plates. Series of sections were then stained with primary antisera against CD68 (AbD

Serotec, Cat. No. MCA1957), GFAP (DAKO, Cat. No. Z0334), Subunit C of mitochondrial ATP synthase (SCMAS; Abcam, Cat. No. ab181243), or cleaved caspase 3 (Asp175) (Cell Signaling, Cat. No. 9661S) followed by either rabbit anti-rat (VectorLab) or swine anti-rabbit (DAKO) secondary antibodies, and immunoreactivity detected with Vectastain ABC (avidin-biotin) kit (Vector) and diaminobenzidine as a chromogen.

*Quantitative microscopy analyses*

Non-overlapping images were captured, on three consecutive sections, through each region of interest. All RGB images were captured via a live video camera (JVC, 3CCD, KY-F55B), mounted onto a Zeiss Axioplan microscope using a x 40 objective and saved as JPEGs. All parameters including lamp intensity, video camera set-up and calibration were maintained constant throughout image capturing. For quantification of storage and inflammation, images were analyzed using ImageJ analysis software (NIH), using an appropriate threshold that selected the foreground immunoreactivity above background. This threshold was then applied as a constant to all subsequent images analyzed per batch of animals and reagent used to determine the specific area of immunoreactivity for each antigen in each region. Data were plotted graphically as the mean percentage area of immunoreactivity per field ± s.e.m. for each region. For quantification of cell death, images were analyzed manually and scored for the presence of apoptotic nuclei. Data were plotted graphically as the mean number of apoptotic cells per field ± s.e.m. for each region. All analyses were performed blind to genotype and treatment.

*Bioanalytical Methods for Analysis of In-Vivo Study Samples*

LC-MS/MS bioanalytical methodologies were developed for the analysis of Trehalose, Miglustat and glucose in mouse whole blood and brain.

Blood samples were prepared as follows; 20 μL of whole blood was transferred to a 96 wellplate and diluted 1:1 with sterile distilled water. Samples were protein precipitated by addition of 160 μL of 2.5 % of trichloroacetic acid (TCA). At the time of analysis, 1 volume of acetonitrile containing an internal standard was added in order to match the initial chromatography conditions, samples were centrifuged at 4350 rpm for 10 min at ambient temperature, and the resultant supernatant transferred for analysis on the LC-MS/MS.

Tissue samples were homogenized in 8 volumes of sterile distilled water and 1 volume of TCA (20% in water) to generate a final concentration of 2% TCA, homogenates were centrifuged at 13000 rpm for 15 min at 4°C and the supernatant transferred to a fresh plate. At the time of analysis, 1 volume of acetonitrile containing an internal standard was added in order to match the initial chromatography conditions, samples were centrifuged at 4350 rpm for 10 min at ambient temperature, and the resultant supernatant transferred for analysis on the LC-MS/MS.

II. RESULTS

*Example 1: Inhibition of trehalase by Miglustat*

Based on reports indicating that the iminosugar miglustat inhibits the activities of several mammalian disacchar-

idases, it was investigated whether miglustat also inhibits trehalase activity. To this aim, an in vitro colorimetric assay was performed to measure the rate of generation of glucose, the product of trehalose hydrolysis by trehalase. In the absence of miglustat, trehalose was rapidly hydrolysed by purified trehalase, and the glucose produced could be quantified at 340 nm absorbance. In the test conditions, the amounts of glucose detected increased over time to reach a plateau in ~20 mins. In contrast, in the presence of miglustat, only a minimal amount of glucose was generated, similar to a control in which trehalase was not added to the reaction (not shown). Thus, miglustat effectively inhibits the trehalase-mediated hydrolysis of trehalose to glucose.

*Example 2: Effect of orally administered Trehalose, Miglustat, and a combination of Trehalose and Miglustat on cell death in Batten mice.*

Brain atrophy is among the hallmarks of Batten disease and is the result of relentless neurodegeneration (Weimer et al, 2009). $Cln3^{-/-}$ mice recapitulate this feature, which can be observed as a global loss of brain weight with age, or decreased neuronal number in various regions including the thalamus, cerebellum, and the somatosensory regions of the cortex. To quantify the extent of neuronal cell death in the test and control mouse groups, immunohistological staining of 10- month-old brain sections was performed using an antibody against cleaved caspase-3, a marker of programmed cell death, and then manually counted the number of cells per section that were reactive.

This analysis showed that the single trehalose and miglustat treatments were effective in abating programmed cell death in nearly all the regions analyzed, with the combined treatment equaling to or exceeding the effects of each single treatment (Figures 1A-D).

When treated with Trehalose + and - high and low doses of Miglustat (200mg/kg or 600mg/kg) and Miglustat alone, similar results were seen in the ventral posterior medial and the ventral posterolateral thalamic nuclei of WT mice and Trehalose-treated, Miglustat-treated Batten mice, and Batten mice treated with Trehalose plus Miglustat. The results shown in Figure 1E show that the untreated $Cln3^{\Delta ex7\text{-}8}$ mice have increased cell death at 12 months of age, which is partially prevented by Trehalose administration and largely suppressed by Miglustat alone and Miglustat/Trehalose oral administration.

This study demonstrated that Miglustat alone, as well as the combination of Miglustat and Trehalose, are capable of inhibiting neuronal cell death and reducing neuroinflammation in a mouse model of CLN3 disease. Cleaved CAS-3 analysis in the VPM/VPL region demonstrate that untreated $Cln3_{\Delta ex7\text{-}8}$ mice experience increased percentages of cell death at 12 months of age over wild type animals. Disease-specific cell death is partially reduced by access to 2% Trehalose in drinking water and prevented by the administration of miglustat alone or of the combination Trehalose and Miglustat.

*Example 3: Effect of orally administered Trehalose, Miglustat alone, and a combination of Trehalose and Miglustat on neuro-inflammation and cellular waste accumulation.*

Chronic neuroinflammation is observed in both patients with CLN3 disease and nonclinical animal models. Data from animal models of CLN3 disease demonstrate early signs of glial activation that precedes neuronal loss (Pontikis et al., 2005). Inhibition of neuroinflammation can significantly attenuate axonal damage, neuron loss, retinal thinning, and brain atrophy in the CLN3 disease animals, suggesting that neuroinflammation may provide important extrinsic signals that influence neuronal function and survival during the disease (Groh et al., 2017).

One potential mechanism for Trehalose and Miglustat in the treatment of CLN3 disease is the reduction of neuroinflammation (microglial activation and astrogliosis), and subsequent amelioration of cell apoptosis within various areas of the CNS. The present study investigated the role of these drugs in reducing neuroinflammation in a mouse model of CLN3 disease.

Neuroinflammation (measured by the number of GFAP-positive astrocyte cells in the neuronal system), and microglia activation into the nervous system (measured by an increase in CD68 staining) were evaluated in wild-type and CLN3-deficient mice.

- Microglial activation (CD68 staining)

$Cln3^{-/-}$ mice display astrocytosis and microglial activation in brain regions where neuronal loss is subsequently most pronounced. These regions include thalamic nuclei that relay somatosensory information to the primary somatosensory cortex (ventral posterior medial and lateral nuclei, VPM/VPL), the somatosensory barrelfield cortex (S1BF), the primary visual (V1) cortex, and the dorsolateral geniculate (DLG) (Pontikis et al., 2005). To quantify microglial activation, antibodies against the microglial marker CD68 were used. Following immunohistochemical (IHC) staining, 5-6 images were acquired per region/mouse (corresponding to 40-50% of region) by a brightfield scanner and quantified microglial activation by threshold analyses using ImageJ Analysis software (NIH).

As expected, the analyzed regions of $Cln3^{-/-}$ mice displayed significantly increased microglial activation compared to WT mice, which was partially mitigated by trehalose administration (Figure 2A-D).

Administration of miglustat alone potently prevented microglial activation in the VPM/VPL and significantly decreased microglial activation in the DLG. Administration of even low concentrations of Miglustat largely reduce CD68 staining in the VPM/VPL (Figure 2 E) and partially or largely reduce CD68 staining in the cerebellum of the mice (not shown).

Combined trehalose/miglustat treatment resulted in a more effective prevention of microglial activation in the DLG and no additional differences compared to either single treatment in the other regions. Indeed, in the VPM/VPL, mice treated with miglustat alone or with the trehalose/miglustat combination were undistinguishable from WT mice (Figure 2A-D).

- Neuroinflammation (GFAP staining)

In addition to increased microglial activation, previous reports describe clusters of GFAP+ astrocytes with extended reactive processes across the cerebellum with disordered Purkinje cell processes and the loss of Purkinje cells themselves.

Microglial activation was also quantified by performing immunohistochemistry staining of brain sections of all test and control mice using anti-GFAP antibodies. Untreated $Cln3^{-/-}$ mice displayed severe astrocytosis in multiple regions of the brain, including the VPM/VPL, DLG, S1BF, and V1 as previously reported (Pontikis et al., 2005).

The results of the single and combined treatments varied regionally: in the VPM/VPL, trehalose alone did not have any observable effects on astrocytosis, whereas miglustat (alone or in combination with trehalose) resulted in levels of GFAP immunoreactivity undistinguishable from that of WT mice (Figure 3A). In the DLG, both trehalose and miglustat as single therapies prevented astrocytosis in $Cln3^{-/-}$ mice, and this reduction in GFAP immunoreactivity was even more significant in the combination treatment group (Figure 3B). In the S1BF, miglustat (but not trehalose) treatment resulted in a reduction in astrocytosis, although this did not reach statistical significance (Figure 3C). Finally, neither treatment led to a substantial change in astrocytosis within V1 (Figure 3D).

To sum-up, the analysis of astroglial activation (GFAP staining) showed that the untreated $Cln3^{\Delta ex7-8}$ mice had increased astrocytosis in the VPM/VPL region at 12 months of age, which was partially prevented by Trehalose administration and largely suppressed by Miglustat alone (even at low doses) and by Miglustat/Trehalose oral administration (Figure 3E).

The beneficial effect of Miglustat on the neuroinflammation and microglial activation in mice having a CLN3 mutation can be explained by the fact that Miglustat reversibly inhibits glucosylceramide synthase, which catalyses the first committed step of glycosphingolipid synthesis. Due to this activity, Miglustat has been successfully used for treating lysosomal storage disorders involving abnormal accumulation of gangliosides.

However, until recently, the Batten disease was thought not to trigger such abnormal accumulation of gangliosides: on the contrary, it was shown in 2018 that the overall levels of lactosylceramides and glycosphingolipids were actually decreased in CLN3-defective cerebellar cells (Somogyi et al, 2018; Schmidtke et al, 2019). This could have precluded the use of Miglustat in CLN3.

Yet, by assessing the changes in the accumulation of harmful quantities of GM2 gangliosides in the Cerebellum by immunodetection of subunit C of ATPase (a major component of juvenile Batten inclusion bodies in the Cerebellum). and the aberrant lysosomal storage (by SCMAS staining) it is now apparent that the untreated $Cln3^{\Delta ex7-8}$ mice have increased ganglioside storage in the cerebellum at 12 months of age (see example 5 below).

Importantly, administration of Trehalose partially reduces this increased storage, whereas Miglustat has no consistent effects on said storage levels. Besides, simultaneous administration of Trehalose and Miglustat consistently obtains the best clearance effects.

Previously thought of as minimal and therefore not harmful, recent evidence suggests that abnormal accumulation of harmful gangliosides may be also reduced by daily doses of Miglustat, because this molecule is a known inhibitor of glycosphingolipid synthesis in the brain and is able to decrease the erroneous accumulation of harmful quantities of GM2 gangliosides, in other diseases such as Gaucher disease.

Importantly, Miglustat does not reduce the total level of ganglioside and globosides in CLN3 mutated cells, but in fact affects the transport of these gangliosides within the cells (see example 5 below).

*Example 4: Pharmacokinetic and pharmacodynamic results for administration of oral and intravenous Trehalose.*

Due to the poor PO bioavailability of Trehalose, it was proposed to use IV dosing of Trehalose in the clinic alongside PO doses of Miglustat. A study was conducted in mouse with PO and IV doses of Trehalose conducted with and without PO doses of Miglustat.

The purpose of this example was to compare the blood PK of unlabelled high doses of Trehalose after IV and PO

administration, with and without Miglustat PO dosing. For the IV comparison, Trehalose was dosed as an IV bolus dose 15 min after PO dosing of Miglustat. For the PO comparison Miglustat was dosed simultaneously with Trehalose. Fasted male C57BL6/N mice were dosed as detailed in Table III:

| Group | Route | Dose | Formulation | n |
|---|---|---|---|---|
| 1 | IV | 1 g/kg trehalose | Sterile distilled water 200 mg/mL trehalose | 4 |
| 2 | PO/IV | 150 mg/kg miglustat PO & 1 g/kg trehalose IV | Sterile distilled water 15 mg/mL miglustat (PO) & 200 mg/mL trehalose (IV) | 4 |
| 3 | PO | 10 g/kg trehalose | Sterile distilled water 1g/mL trehalose | 4 |
| 4 | PO | 10 g/kg trehalose & 150 mg/kg miglustat | Sterile distilled water lg/mL trehalose & 15 mg/mL miglustat | 4 |

Blood samples were collected at pre-dose, 10, 20, 30, 60, 120 and 180 min post-dose for group 1 and 2 and at pre-dose, 15, 30, 45, 60, 120 and 180 min post-dose for group 3 and 4.

It is important to notice that the Trehalose dose for oral administration is ten times higher than the dose for IV injection. This is because low doses of Trehalose such as 1g/kg cannot be detected after oral administration.

Blood concentrations of Trehalose were analysed with LC-MS/MS and PK parameters are summarized in Table IV:

| Dose (g/kg) & Route | $C_{max}$ (μM) | $T_{max}$ (min) | $AUC_{0-inf}$(μM.min) | Trehalose fold-change with miglustat dosing |
|---|---|---|---|---|
| 1 IV | 3689.2 ± 152.7 | 20 | 117389.3 ± 20735.8 | - |
| 1 IV + miglustat | 5494.3 ± 873.3 | 20 | 179501.0 ± 17152.8 | 1.5 |
| 10 PO | 148.4 ± 73.2 | 15 | 11170.5 ± 3541.6 | - |
| 10 PO + miglustat | 188.6 ± 43.9 | 15 | 18394.3 ± 4560.5 | 1.6 |

The $C_{max}$ results clearly show that oral administration of Trehalose is much less efficient than IV administration, as the level of Trehalose in blood is 25 folds less even if 10 times more of Trehalose has been administered.

The results moreover show that the combined administration of PO Miglustat with Trehalose *via* either IV or PO administration significantly increased the measured AUC of Trehalose (>1.5 fold). Yet, the administration of Miglustat does not enhance the level of PO Trehalose to a sufficient level, even if 10 times more Trehalose is administered: the $C_{max}$ results clearly show that combination of oral administration of Trehalose and Miglustat is still much less efficient than when Trehalose is administered by IV (with or without Miglustat), as the level of Trehalose in blood is 29 folds less even if 10 times more of Trehalose has been administered.

Tissue concentrations of Trehalose were analysed with LC-MS/MS and PK parameters are summarized in Table V :

| Dose (g/kg) & Route | Concentration (nmol/g) Liver | Concentration (nmol/g) Brain R | Concentration (nmol/g) Brain L |
|---|---|---|---|
| 1 IV | 167.9 = 24.6 | 7.3 = 0.9 | 6.9 ± 1.9 |
| 1 IV + miglustat | 278.6 = 96.6 | 10.5 = 1.7 | 10.8 ± 2.0 |
| 10 PO | 191.0 = 21.2 | 2.6 = 1.2 | 2.5 ± 1.0 |
| 10 PO + miglustat | 142.7 = 30.2 | 3.3 = 1.2 | 3.3 ± 1.2 |

Tissue levels of Trehalose were above the limit of detection in all analysed samples. To assess variability in sample preparation right and left brain hemispheres were analysed separately demonstrating high reproducibility and low variability.

The results clearly show that administration of Trehalose *via* the IV route led to higher levels in the brain compared to PO dosing (again, although 10 times less Trehalose was administered). Also, the results show that, in combination with oral Miglustat, the brain exposure of Trehalose is significantly increased when measured at 180 min post-dose (from about

7 to about 10 nmol/g). This was surprising that the greatest brain exposures were achieved through IV compared to oral dosing (about 7 nmol/g by IV versus 2.5 nmol/g by PO). This demonstrates that intravenous administration of Trehalose could lead to a significant increase in brain exposure, despite what was described in the art.

It was also demonstrated that oral administration of Miglustat (150 mg/kg) increased Trehalose exposure in brain tissue after oral and IV administration of Trehalose and that IV administration of Trehalose continues to provide the greatest exposures in brain tissue at the same doses. It has been found that, surprisingly, combining Miglustat with oral Trehalose does not increase plasma or brain tissue exposure to the same extent as for the combination or miglustat with IV trehalose.

Thus, only IV administration of Trehalose combined with oral application of Miglustat can significantly increase Trehalose brain tissue exposure in WT C57BL/6 mice. Without being bound to this theory, this effect is possible due to the fact that Miglustat can not only prevent Trehalose from being hydrolysed by the Trehalase enzyme present in the plasma, but it can also stabilize Trehalose in the brain. Miglustat crosses the blood-brain barrier and has been shown to be distributed to the brain.

*Example 5: Lysosomal storage of glycosphingolipids in cellular and zebrafish models of CLN3 disease can be treated with miglustat*

Glycosphingolipids (GSLs) are a critical family of lipids that act as structural membrane components, cell surface receptors, immune receptors and as signalling molecules. GSLs are critical components of the myelin sheath and are modulators of growth factor and $Ca^{2+}$ ion signalling, ensuring that they have critical roles in brain development and function. Indeed, an absence of GSLs in mice genetically null for the ceramide glucosyltransferase (CGT) enzyme that catalyses the first step in GSL biosynthesis is associated with early lethality within the first two weeks of life (Jennemann et al, 2005). Furthermore, abnormal GSL degradation, which occurs within the lysosome, is associated with a family of human neurodegenerative and multi-systemic diseases called lysosomal storage disorders (LSDs) (Ryckman AE et al, 2020). Reducing GSL accumulation in LSDs (known as substrate reduction therapy or SRT), namely in Gaucher disease, which is the most common individual LSD with an incidence of 1:20,000, is an approved therapeutic strategy in Europe and the US. By partially inhibiting GSL production via inhibition of CGT it is possible to reduce the volume of GSL entering the lysosome and in some cases where there is residual enzyme activity within the lysosome it is possible to even slowly reduce lysosomal GSL storage content (Platt FM et al, 2008).

CLN3 disease has previously been associated with both the accumulation of certain GSLs in patient post-mortem tissue and also with reduced levels of certain specific GSLs, namely ganglioside GM1, in cell lines (Somogyi et al, 2018). It was therefore decided to investigate the localisation and levels of certain GSLs, namely neutral globosides and acidic gangliosides in a cohort of CLN3 disease cell lines and a morpholino generated zebrafish model. Based on the results showing that there is indeed abnormal lysosomal localisation and lysosomal storage of certain GSLs in CLN3-impaired cells, the capability of an inhibitor of CGT (miglustat) to reduce global GSL levels, and most importantly to normalise GSL localisation and correct function in human cells and in a fish model.

**5.1. Materials and Methods:**

*Fibroblasts*: Human fibroblasts were from Coriell Cell Repository (GM05399, apparently healthy control) in which CRISPR/Cas9 was used to generate the CLN3 1kb mutant line. Cells were maintained in DMEM with 10% foetal bovine serum (FBS) and 1% L-glutamine (no antibiotics) and grown as monolayers in 5% CO2 at 37°C in a humidified incubator. For all experiments cells were passage matched and not used beyond passage 25.

*iPSCs*: Human CLN3 (homozygous for the common 1kb deletion) and age matched control KOLF2 neuronal progenitor cells (NPCs) were generated, maintained in mTesR medium and then differentiated into mature cortical neurons according to the protocols described in Kemp P.J. et al, 2016.

*Miglustat treatment*: all cells were treated with miglustat (100mM stock in mQ $H_20$) at a concentration of 50μM for 7 days. This effective concentration and incubation time in cells is well characterised in the literature (Platt et al, JBC 1994, Te Vruchte et al, 2004).

*Preparation for microscopy*: For all fluorescent imaging experiments, cells were grown in coated Ibidi 8 well chamberslides in the indicated medium above, for NPC-derived neurons the chamberslides were first coated with Matrigel to ensure adherence to the chamber surface. Adherent cells were grown in the chambers overnight prior to staining and imaging whereas neurons were plated as NPCs onto Matrigel coated chambers and were differentiated within the wells for 21 days as described above (maturity confirmed by ePhys[5]) with miglustat treatment occurring over the last 7 days.

*Breeding and maintenance of Zebrafish strains*: Zebrafish (TL strain) were purchased from University College London and maintained as breeding stock at the Cardiff University aquarium in temperature controlled (28°C) tanks of an automated housing system in aquarium grade water supplemented with methylene blue (0.0002%) and fed twice daily. Prior to fixation and wholemount fluorescence staining all fish were euthanised by terminal anaesthesia using MS222 (0.16% w/v). All procedures were performed in accordance with the UK Home Office Animals Scientific Procedures Act

(1986). Morpholino's (MOs), for oligonucleotide knockdown of cln3, were designed and manufactured by Gene Tools (Philomath, OR, USA) with the cln3ATG morpholino published in Wager et al, 2016, used in this study (5'-CATTGCG ACTTTCACAGGAGAAATG-3', SEQ ID NO:1). A 1mM stock solution was generated by re-suspension of lyophilized solid in mQ $H_2O$ followed by heating at 65°C for 10min. MOs were diluted as necessary and injected into the yolk of embryos at the 1-2 cell stage by lining embryos up against a microscope slide in a petri dish and injecting a 2μl volume of MO into the embryo via a 1mm OD x 0.58mm ID borosilicate glass needle using a Narishige micromanipulator. Embryos were maintained until 96 hours post fertilisation (hpf) in aquarium water with a miglustat treatment group switching to aquarium water with 300μM miglustat from 24hpf.

*Wholemount immunofluorescence*: Larvae were anaesthetised then fixed in 4% (w/v) paraformaldehyde in phosphate buffered saline overnight at 4°C. Subsequently, larvae were washed in PBS and then incubated with fluorescent lipid markers (cholera toxin, see below) or anti-SCMAS antibodies as outlined below. All imaging was performed wholemount using a Zeiss lightsheet microscope as outlined below.

*Glycosphingolipid immunochemistry*: Glycosphingolipid (GSL) localisation and levels were determined using glycolipid specific Shiga Like toxin 1 (SLxT, Cambridge Bioscience) and Alexa Fluor 488 or 595 labelled cholera toxin B (CtxB) subunit (Invitrogen) toxins against the neutral GSL globoside (Gb3 and Gb4 sub-species) and the acidic ganglioside GM1 respectively. Lipids were imaged in paraformaldehyde (4%, 10min) fixed cells or zebrafish (fixed overnight) by overnight staining at 4°C with a final concentration of 1μg/ml of both toxins in Dulbecco's modified phosphate buffered saline (DPBS) supplemented with 1% bovine serum albumin and 0.1% saponin. Samples were washed 3x in DPBS and then immediately counter stained with nuclear marker (Hoechst, 5μg/ml in DPBS) in the case of fluorescently tagged CtxB or with the SLxT labelled cells first incubated with anti-SLxT primary antibody (1:500, Cambridge Bioscience) followed by fluorescent secondary antibody (both: 1:200 dilution, 1h, room temperature, Abcam) with 3x DPBS washes after each incubation prior to nuclear counter stain.

*Imaging*: Unless otherwise indicated, all cells were imaged on a widefield single wavelength excitation LED fluorescence Zeiss Axiovert microscope with a high speed monochrome Zeiss MRm CCD camera. Images were taken with Zeiss Axiovision 4.8.1 software and post-processed with Photoshop CS6 and ImageJ. For co-localisation studies, images of fixed and stained cells were taken using a Leica TCS SPE confocal system with the Leica LASX software and images subsequently analysed using ImageJ. For zebrafish embryos, all imaging was done on whole fixed embryos mounted in agar on the Zeiss Lightsheet microscope with Zen software for image capture and deconvolution.

*Electron microscopy (EM):* Fibroblast cells were grown in T75 flasks until confluent, were washed, removed from the growth surface by mild trypsin digestion and were harvested and washed 3 times by centrifugation (700g). Cell pellets were re-suspended in EM fixative (0.5% glutaraldehyde in 0.2M HEPES buffer, pH 7.2) for 20min and then washed, pelleted and maintained in EM buffer prior to post-fixation in 0.1M sodium cacodylate for 30min and subsequent addition of 1.5% potassium ferrocyanide and 1% osmium tetroxide for a further 30min. Pellets were dehydrated through an ethanol series (10min per wash) prior to incubation with propylene oxide (1h) and embedding in 100% Durcupan ACM embedding mixture and polymerisation at 60-70°C. Ultra-thin sections of 50nm thickness were cut with a Reichert OMU4 ultramicrotome stained with lead citrate for 10min and then imaged using a JEOL 1200EX transmission electron microscope fitted with an Orius 200 digital camera (Gatan, Abingdon UK).

*Data analysis:* All data was analysed using Graphpad Prism 6.

## 5.2. Results

### *5.2.1. Glycosphingolipid storage in human CLN3 disease fibroblasts*

The GSL storage was observed in CLN3 1kb deletion mutant skin fibroblasts. As can be seen on Figure 4B, there is a noticeable accumulation of both gangliosides GM1 and globosides Gb3/4 in punctate perinuclear lysosomes in the CLN3 mutant cells compared to low levels of plasma membrane staining of CtxB in the CLN3 cells compared to the untreated control human cells.

Treatment with 50μM miglustat for 5 days led to a significant reduction in the accumulating GSLs back to control levels and a normalisation in localisation of CtxB back to the plasma membrane. It is noteworthy that in the mutant fibroblasts, we did not observe the lower overall levels of ganglioside GM1 that were described previously (Somogyi et al, 2018), suggesting there is no biosynthesis defect of these gangliosides in human cells.

In addition to GSL, the effect of miglustat was determined on the storage of other molecules in the CLN3 disease, including the classical storage of SCMAS. As can be seen on Figure 4B, there is substantial accumulation of SCMAS in CLN3 mutant human fibroblasts and this accumulation is significantly reduced in the miglustat treated cells. This miglustat induced reduction in lysosomal storage of multiple macromolecules is in line with an overall improvement in lysosomal health as illustrated by a normalisation in the visible expansion of the lysosomal system in the CLN3 cells (normalisation of lysotracker staining, Figure 4B/C) and a normalisation in elevated autophagy, as measured using the live cell autophagy marker CytoID.

The reduction in autophagy may also be aligned to improved mitochondrial function, which was observed by electron microscopy (Figure 5), where swollen mitochondria with visible abnormalities in their cristae in the CLN3 mutant cells are normalised by miglustat. These improvements in organelle function are also accompanied by improvements in cellular $Ca^{2+}$ stores. The elevated lysosomal $Ca^{2+}$ that was measured previously in CLN3 disease (Chandrachud U. et al, 2015) is restored to healthy control levels following miglustat treatment (Figure 4A). Together, these data indicate that miglustat not only reduces GSL storage but in doing so also normalises organelle health, function, cellular signalling and restores normal SCMAS protein processing within the lysosome.

5.2.2. *GSLs accumulate within lysosomes in CLN3 mutant cells*

To confirm that the accumulating macromolecules observed in the CLN3 human mutant line were indeed GSLs accumulating within lysosomes, confocal and electron microscopy were used (Figure 5). It was initially determined whether the accumulating CtxB bound to ganglioside GM1 co-localised with the lysosomal soluble protein marker NPC2 (Figure 5A).

As can be seen from the indicated correlations, there is no clear co-localisation between CtxB-ganglioside GM1 and NPC2 in the control cells, whereas in the CLN3 mutant there is both a clear overlap between CtxB and NPC2 and reduced levels at the cell periphery (plasma membrane) indicative of lysosomal storage caused by redistribution respectively. Miglustat treatment not only clearly reduces the peak CtxB fluorescence, indicating the reduction in GSL biosynthesis via CGT inhibition, but also restores sub-plasma membrane localisation indicative of a normalisation of GSL trafficking. By EM human control cell lysosomes (asterisks) are either spherical lightly stained electron dense objects or are spherical clear objects with darker material appearing as clumps around the edges (Figure 5B, labelled inset) whereas in the CLN3 disease mutant cells the presence of multiple dark membranous inclusions resembling whorls of lipid (indicative of either globosides or gangliosides) or lysosomes containing floccular material indicating the presence of protein/peptide accumulation can be seen.

Following miglustat treatment (Figure 5B), it was still possible to observe the expanded lysosomal system but the individual lysosomes now appear as 'empty' lysosomes within which there is the presence of peripheral dark clumped material as observed in the control cells and a notable absence of the dark membranous whorl-like inclusions observed in the untreated CLN3 cells. These data are indicative of a clear lysosomal accumulation of GSLs, that can be cleared by miglustat treatment and support the notion that reducing GSL biosynthesis allows the clearance of lysosomal storage.

*5.2.3. Miglustat reduces glycosphingolipid storage in human CLN3 disease NPC derived cortical neurons*

Having demonstrated and confirmed the presence of GSL storage in CLN3 mutant skin fibroblasts by microscopy and EM, it was next determined the presence of GSL storage in human CLN3 disease neurons. Cortical neurons were prepared from NPCs using differentiation protocols characterised and published by Kemp PJ. et al 2016.

First, iPSCs were differentiated into neuronal progenitor cells in mTeSR medium with appropriate supplements followed by a 4-week differentiation period into cortical neurons using a high extracellular $Ca^{2+}$ synapto juice A and B protocol. Miglustat (50$\mu$M) was added during the final week of differentiation. A prominent accumulation of globoside, rather than ganglioside GM1 was observed (Figure 6A), although plasma membrane staining with CtxB was reduced in all cases. Lipid accumulation was reflected by the increased lysosomal expansion shown by increased lysotracker staining intensity. Lysosomal protein storage was demonstrated by the presence of punctate SCMAS storage alongside elevated autophagy, which is the likely source of this protein (Figure 6A). In all cases, miglustat treatment to reduce GSL biosynthesis led to reduced lipid and protein storage levels, normalisation of lipid localisation (Golgi localisation in the treated CLN3 neurons compared to punctate lysosomes in the untreated CLN3 cells) and reduction in expansion of the lysosomal and autophagic vacuole systems demonstrated by lysotracker and cytoID staining back to control levels (Figure 6B). In addition, reduced GSL content following miglustat treatment led to a normalisation of excitotoxic glutamate induced $Ca^{2+}$ signalling, with a clear reduction in spontaneous $Ca^{2+}$ spikes and a return to baseline $Ca^{2+}$ levels (as with control) in the miglustat treated human CLN3 mutant neurons. This was accompanied by a normalisation of lysosomal $Ca^{2+}$ content, measured using ionomycin to clamp non-lysosomal $Ca^{2+}$ stores and GPN to burst lysosomes and allow cytosolic measurement of released $Ca^{2+}$ using Fura 2,AM (Figure 6C).

*5.2.4. Miglustat reduces glycosphingolipid storage and normalises eye phenotypes in cln3 morphant zebrafish embryos.*

Zebrafish was used as the model organism in which to test the *in vivo* efficacy of miglustat as a treatment for CLN3 owing to the documented presence in these fish of key CLN3 disease phenotypes (seizures, impaired motor and cognitive function, presence of lipofuscin storage, Wager K. et al. 2016). The present analysis by wholemount lightsheet microscopy confirms the presence of SCMAS accumulation in the *cln3* morphant (*cln3$^{morph}$*) fish as previously reported (Figure 7 and

Wager K. et al 2016). In addition, the presence of glycolipid storage (and an increase in levels) was also observed, including ganglioside GM1 by cholera toxin (CtxB). Notably, miglustat treatment (300μM, a high concentration needed to penetrate the chorion) from 4hpf for 88h leads to a decrease, particularly in the brain, in fluorescence intensity of both glycolipids and of lipofuscin associated SCMAS storage in the *cln3$^{morph}$* zebrafish. This can be seen on Figure 7C from the associated line scans across the brain (from eye to eye, with bright GSL enriched eye areas indicated by arrows) where clear focal spots indicative of ganglioside GM1 storage within the brain can be seen in the cln3 morphant fish (grey line) which is normalised following miglustat treatment (thin black line). The impact of miglustat on reported zebrafish developmental phenotypes, including retinal area, is also indicated as smaller retinas in the cln3 morphant are restored to wild-type levels (despite the overall reduction in GSL biosynthesis, demonstrating the considerably more damaging impact of lysosomal GSL storage).

*Conclusion*

In conclusion, it has been identified that the combined treatment of miglustat and trehalose is a potential treatment for Batten disease. The present results also indicate that miglustat exerts its beneficial effects according to at least two distinct modalities of action. First, miglustat reduces the activity of the trehalase, thereby potentially increasing the bioavailability of trehalose and its favorable effects on the autophagy-lysosome system. Second, miglustat has a clear modifying action on *Cln3$^{-/-}$* mouse neuropathology that is independent of, and significantly stronger than, that of trehalose alone.

The potent neuroprotective effects exerted by miglustat suggest that certain biochemical products of the glycosphingolipid pathway may also accumulate in Batten disease and contribute to its pathogenesis. Additional studies have now clarified the link between CLN3 deficiency and the mechanism of action of miglustat, as shown in example 5 above. The present study indicates that trehalose and miglustat can exert both orthogonal and synergistic effects that are beneficial in counteracting the Batten disease progression, thereby laying the ground for optimized treatments of the Batten disease.

**BIBLIOGRAPHIC REFERENCES**

Aberg L, Autti T, Braulke T, Cooper JD, van Diggelen OP, Jalanko A, Kenrick S, Kitzmüller C, Kohlschütter A, Kyttälä A, Mitchison HM, Mole SE, Niezen-de-Boer R, Punkari ML, Schulz A, Talling M, and Williams RE. CLN3. In: Mole SE, Williams RE, Goebel HH, editors. The Neuronal Ceroid Lipofuscinoses (Batten Disease) Oxford University Press; Oxford: 2011, p 110-139.

Bozorg, S., Ramirez-Montealegre, D., Chung, M., and Pearce, D.A. (2009). Juvenile neuronal ceroid lipofuscinosis (JNCL) and the eye. Survey of ophthalmology 54, 463-471.

Butters TD (2007). Pharmacotherapeutic strategies using small molecules for the treatment of glycolipid lysosomal storage disorders. Expert Opin Pharmacother.,8(4):427-35

Cao, Y., Espinola, J.A., Fossale, E., Massey, A.C., Cuervo, A.M., MacDonald, M.E., and Cotman, S.L. (2006). Autophagy is disrupted in a knock-in mouse model of juvenile neuronal ceroid lipofuscinosis. J. Biol. Chem. 281, 20483-20493.

Cárcel-Trullols, J., Kovács, A.D., and Pearce, D.A. (2015). Cell biology of the NCL proteins. What they do and don’t do. Biochimica et biophysica acta 1852, 2242-2255.

Cendret, V., et al (2015). Synthetic deoxynojirimycin derivatives bearing a thiolated, fluorinated or unsaturated N-alkyl chain: identification of potent α-glucosidase and trehalase inhibitors as well as F508del-CFTR correctors. Org Biomol Chem. 2015 Nov 21;13(43).

Chandrachud U, Walker MW, Simas AM, Heetveld S, Petcherski A, Klein M, Oh H, Wolf P, Zhao WN, Norton S, Haggarty SJ, Lloyd-Evans E, Cotman SL (2015) Unbiased Cell-based Screening in a Neuronal Cell Model of Batten Disease Highlights an Interaction between Ca2+ Homeostasis, Autophagy, and CLN3 Protein Function. J Biol Chem. 290(23):14361-80.

DeBosch, B.J., Heitmeier, M.R., Mayer, A.L., Higgins, C.B., Crowley, J.R., Kraft, T.E., Chi, M., Newberry, E.P., Chen, Z., and Finck, B.N., et al. (2016). Trehalose inhibits solute carrier 2A (SLC2A) proteins to induce autophagy and prevent hepatic steatosis. Science signaling 9, ra21.

Dehay, B., Bové, J., Rodríguez-Muela, N., Perier, C., Recasens, A., Boya, P., and Vila, M. (2010). Pathogenic lysosomal depletion in Parkinson’s disease. J.Neurosci. 30, 12535-12544.

Du, J., Liang, Y., Xu, F., Sun, B., and Wang, Z. (2013). Trehalose rescues Alzheimer’s disease phenotypes in APP/PS1 transgenic mice. The Journal of pharmacy and pharmacology 65, 1753-1756.

Fossale, E., Wolf, P., Espinola, J.A., Lubicz-Nawrocka, T., Teed, A.M., Gao, H., Rigamonti, D., Cattaneo, E., MacDonald, M.E., and Cotman, S.L. (2004). Membrane trafficking and mitochondrial abnormalities precede subunit c deposition in a cerebellar cell model of juvenile neuronal ceroid lipofuscinosis. BMC.Neurosci. 5, 57.

Getty, A.L., Pearce, D.A. Interactions of the proteins of neuronal ceroid lipofuscinosis: clues to function (2011). Cell. Mol. Life Sci. 68, 453-474

Groh, J., Berve, K., and Martini, R. (2017). Fingolimod and Teriflunomide Attenuate Neurodegeneration in Mouse Models of Neuronal Ceroid Lipofuscinosis. Molecular therapy : the journal of the American Society of Gene Therapy 25, 1889-1899.

Jennemann R. et al, Cell-specific deletion of glucosylceramide synthase in brain leads to severe neural defects after birth. Proc Natl Acad Sci U S A. 2005 Aug 30;102(35):12459-64

Kang, S., Heo, T.-H., and Kim, S.-J. (2014). Altered levels of $\alpha$-synuclein and sphingolipids in Batten disease lymphoblast cells. Gene 539, 181-185.

Kemp P.J. et al, Improving and accelerating the differentiation and functional maturation of human stem cell-derived neurons: role of extracellular calcium and GABA. J Physiol. 2016 Nov 15;594(22):6583-6594

Khalifeh, M., Barreto, G.E., and Sahebkar, A. (2019). Trehalose as a promising therapeutic candidate for the treatment of Parkinson's disease. Br J Pharmacol 176, 1173-1189.

Lee, HJ., Yoon, YS. & Lee, SJ. Mechanism of neuroprotection by trehalose: controversy surrounding autophagy induction (2018). Cell Death Dis 9, 712

Luiro, K., Yliannala, K., Ahtiainen, L., Maunu, H., Järvelä, I., Kyttälä, A., and Jalanko, A. (2004). Interconnections of CLN3, Hook1 and Rab proteins link Batten disease to defects in the endocytic pathway. Hum Mol.Genet. 13, 3017-3027.

Medina, D.L., Fraldi, A., Bouche, V., Annunziata, F., Mansueto, G., Spampanato, C., Puri, C., Pignata, A., Martina, J.A., and Sardiello, M., et al. (2011). Transcriptional activation of lysosomal exocytosis promotes cellular clearance. Developmental cell 21, 421-430.

Nikoletopoulou, V., Papandreou, M.-E., and Tavernarakis, N. (2015). Autophagy in the physiology and pathology of the central nervous system. Cell death and differentiation 22, 398-407.

Nixon, R.A., and Yang, D.-S. (2012). Autophagy and neuronal cell death in neurological disorders. Cold Spring Harbor perspectives in biology 4.

Ostergaard, J.R. (2016). Juvenile neuronal ceroid lipofuscinosis (Batten disease). Current insights. Degenerative neurological and neuromuscular disease 6, 73-83.

Palmieri, M., Pal, R., Nelvagal, H.R., Lotfi, P., Stinnett, G.R., Seymour, M.L., Chaudhury, A., Bajaj, L., Bondar, V.V., and Bremner, L., et al. (2017a). mTORC1-independent TFEB activation via Akt inhibition promotes cellular clearance in neurodegenerative storage diseases. Nat. Commun. 8, 14338.

Palmieri, M., Pal, R., Nelvagal, H.R., Lotfi, P., Stinnett, G.R., Seymour, M.L., Chaudhury, A., Bajaj, L., Bondar, V.V., Bremner, L., et al. (2017b). Corrigendum: mTORC1-independent TFEB activation via Akt inhibition promotes cellular clearance in neurodegenerative storage diseases. Nat. Commun. 8, 14338.

Perucho, J., Casarejos, M.J., Gomez, A., Solano, R.M., Yébenes, J.G. de, and Mena, M.A. (2012). Trehalose protects from aggravation of amyloid pathology induced by isoflurane anesthesia in APP(swe) mutant mice. Curr Alzheimer Res 9, 334-343.

Platt FM. Et al, N-butyldeoxygalactonojirimycin inhibits glycolipid biosynthesis but does not affect N-linked oligosaccharide processing. J Biol Chem. 1994 Oct 28;269(43):27108-14.

Platt FM et al, Substrate reduction therapy. Acta Paediatr. 2008 Apr;97(457):88-93

Pontikis, C.C., Cella, C.V., Parihar, N., Lim, M.J., Chakrabarti, S., Mitchison, H.M., Mobley, W.C., Rezaie, P., Pearce, D.A., and Cooper, J.D. (2004). Late onset neurodegeneration in the Cln3-/- mouse model of juvenile neuronal ceroid lipofuscinosis is preceded by low level glial activation. Brain research 1023, 231-242.

Portbury, S.D., Hare, D.J., Sgambelloni, C., Perronnes, K., Portbury, A.J., Finkelstein, D.I., and Adlard, P.A. (2017). Trehalose Improves Cognition in the Transgenic Tg2576 Mouse Model of Alzheimer's Disease. Journal of Alzheimer's disease : JAD 60, 549-560.

Radke, J., Koll, R., Gill, E., Wiese, L., Schulz, A., Kohlschütter, A., Schuelke, M., Hagel, C., Stenzel, W., and Goebel, H.H. (2018). Autophagic vacuolar myopathy is a common feature of CLN3 disease. Annals of clinical and translational neurology 5, 1385-1393.

Ryckman AE et al. Metabolism of Glycosphingolipids and Their Role in the Pathophysiology of Lysosomal Storage Disorders. Int J Mol Sci. 2020 Sep; 21(18): 6881.

Sardiello, M., Palmieri, M., Di Ronza, A., Medina, D.L., Valenza, M., Gennarino, V.A., Di Malta, C., Donaudy, F., Embrione, V., and Polishchuk, R.S., et al. (2009). A gene network regulating lysosomal biogenesis and function. Science 325, 473-477.

Sarkar, S., and Rubinsztein, D.C. (2008). Huntington's disease. Degradation of mutant huntingtin by autophagy. FEBS J 275, 4263-4270.

Sarkar, S., Chigurupati, S., Raymick, J., Mann, D., Bowyer, J.F., Schmitt, T., Beger, R.D., Hanig, J.P., Schmued, L.C., and Paule, M.G. (2014). Neuroprotective effect of the chemical chaperone, trehalose in a chronic MPTP-induced Parkinson's disease mouse model. Neurotoxicology 44, 250-262.

Sarkar, S., Davies, J.E., Huang, Z., Tunnacliffe, A., and Rubinsztein, D.C. (2007). Trehalose, a novel mTOR-

independent autophagy enhancer, accelerates the clearance of mutant huntingtin and alpha-synuclein. J. Biol. Chem. 282, 5641-5652.

Schmidtke, C., Tiede, S., Thelen, M., Käkelä, R., Jabs, S., Makrypidi, G., Sylvester, M., Schweizer, M., Braren, I., and Brocke-Ahmadinejad, N., et al. (2019). Lysosomal proteome analysis reveals that CLN3-defective cells have multiple enzyme deficiencies associated with changes in intracellular trafficking. J Biol Chem 294, 9592-9604.

Settembre, C., Di Malta, C., Polito, V.A., Garcia Arencibia, M., Vetrini, F., Erdin, S., Erdin, S.U., Huynh, T., Medina, D., and Colella, P., et al. (2011). TFEB links autophagy to lysosomal biogenesis. Science 332, 1429-1433.

Somogyi A. et al, Altered Expression of Ganglioside Metabolizing Enzymes Results in GM3 Ganglioside Accumulation in Cerebellar Cells of a Mouse Model of Juvenile Neuronal Ceroid Lipofuscinosis. Int J Mol Sci. 2018 Feb 22;19(2):625

Song, W., Wang, F., Savini, M., Ake, A., Di Ronza, A., Sardiello, M., and Segatori, L. (2013). TFEB regulates lysosomal proteostasis. Human molecular genetics 22, 1994-2009.

Taga, T., et al (1972). The crystal and molecular structure of trehalose dihydrate, Acta Cryst B28, 325.

Tanaka, M., Machida, Y., Niu, S., Ikeda, T., Jana, N.R., Doi, H., Kurosawa, M., Nekooki, M., and Nukina, N. (2004). Trehalose alleviates polyglutamine-mediated pathology in a mouse model of Huntington disease. Nat. Med. 10, 148-154.

Te Vruchte D. et al, Accumulation of glycosphingolipids in Niemann-Pick C disease disrupts endosomal transport. J Biol Chem. 2004 Jun 18;279(25):26167-75

Tien, N.T., Karaca, I., Tamboli, I.Y., and Walter, J. (2016). Trehalose Alters Subcellular Trafficking and the Metabolism of the Alzheimer-associated Amyloid Precursor Protein. J Biol Chem 291, 10528-10540.

Uchida, K., Unuma, K., Funakoshi, T., Aki, T., and Uemura, K. (2014). Activation of Master Autophagy Regulator TFEB During Systemic LPS Administration in the Cornea. Journal of Toxicologic Pathology 27, 153-158.

Uusi-Rauva, K., Luiro, K., Tanhuanpää, K., Kopra, O., Martín-Vasallo, P., Kyttälä, A., and Jalanko, A. (2008). Novel interactions of CLN3 protein link Batten disease to dysregulation of fodrin-Na+, K+ ATPase complex. Exp Cell Res 314, 2895-2905.

Wada S, Sawa R, Ohba S, Hayashi C, Umekita M, Shibuya Y, Iijima K, Iwanami F, Igarashi M (2016). Stability and Bioavailability of Lentztrehaloses A, B, and C as Replacements for Trehalose. J Agric Food Chem., 64(38):7121-6

Wager K. et al Neurodegeneration and Epilepsy in a Zebrafish Model of CLN3 Disease (Batten Disease) PLoS One. 2016 Jun 21;11(6):e0157365.

Weimer, J. M., Benedict, J. W., Getty, A. L., Pontikis, C. C., Lim, M. J., Cooper, J. D., & Pearce, D. A. (2009). Cerebellar defects in a mouse model of juvenile neuronal ceroid lipofuscinosis. Brain research, 1266, 93-107

Zhang, X., Chen, S., Song, L., Tang, Y., Shen, Y., Jia, L., and Le, W. (2014). MTOR-independent, autophagic enhancer trehalose prolongs motor neuron survival and ameliorates the autophagic flux defect in a mouse model of amyotrophic lateral sclerosis. Autophagy 10, 588-602.

## Claims

1. Miglustat for use for reducing ganglioside accumulation and inflammation in neuronal cells patients suffering from the Juvenile Neuronal Ceroid Lipofuscinosis (JNCL disease).

2. Miglustat for use according to claim 1, wherein it is used for reducing neuronal cell death, neuroinflammation and microglial activation in the cerebellum of patients suffering from the Juvenile Neuronal Ceroid Lipofuscinosis (JNCL disease).

3. Miglustat for use according to claim 1 or 2, wherein it is administered orally.

4. Miglustat for use according to any of claims1 to 3, wherein it is administered at a dose ranging from 100 to 600mg per day.

5. Miglustat for use according to any of claims 1 to 4, wherein it is administered to said JNCL patient together with Trehalose, but in a separated manner.

6. Miglustat for use according to any of claims 1 to 4, wherein it is administered as single active principle.

Figure 1

A
Thalamus
VPM/VPL
Cleaved caspase 3
immunoreactivity
(positive cells/area)
0
5
10
15
20
*
*
**
WT
untreated
treh
mig
treh+mig
Cln3−/−
B
Thalamus
dLG
Cleaved caspase 3
immunoreactivity
(positive cells/area)
0
1
2
3
4
***
***
WT
untreated
treh
mig
treh+mig
Cln3−/−

C

Cleaved caspase 3
immunoreactivity
(positive cells/area)
0
1
2
3
WT
untreated
treh
mig
treh+mig
Cln3−/−
Cortex
S1BF

Figure 1 (continued)

D

Cleaved caspase 3
immunoreactivity
(positive cells/area)
0
1
2
3
4
5
WT
untreated
treh
mig
treh+mig
Cln3−/−
Cortex
V1
***
***

Figure 1 (continued)

E

No. of CASP3 positive cells/area
14
12
10
8
6
4
2
0
WT - + - - + Trehalose
- - L H L Miglustat
JNCL

A

Immunoreactivity (%)
0
2
4
6
8
WT
untreated
treh
mig
treh+mig
Cln3−/−
**
***
**

B

Immunoreactivity (%)
0.0
0.5
1.0
1.5
2.0
WT
untreated
treh
mig
treh+mig
Cln3−/−
**
*

Figure 2

C

Immunoreactivity (%)
0.0
0.5
1.0
1.5
2.0
WT
untreated
treh
mig
treh+mig
Cln3−/−
***
*

Figure 2 (continued)

D

Immunoreactivity (%)
0
1
2
3
WT
untreated
treh
mig
treh+mig
Cln3−/−
***

Figure 2 (continued)

E

5.0
4.0
3.0
2.0
1.0
0.0
WT
- + - - + Trehalose
- - L H L Miglustat
JNCL

Figure 3

A

Immunoreactivity (%)
15
10
5
0
**
**
*
WT
untreated
treh
mig
treh+mig
Cln3−/−

Figure 3 (continued)

B

Immunoreactivity (%)
2.0
1.5
1.0
0.5
0.0
**
*
**
WT
untreated
treh
mig
treh+mig
Cln3−/−

C

Immunoreactivity (%)
5
4
3
2
1
0
*
WT
untreated
treh
mig
treh+mig
Cln3−/−

Figure 3 (continued)

D

Immunoreactivity (%)
0.0
0.5
1.0
1.5
2.0
WT
untreated
treh
mig
treh+mig
Cln3−/−

E

0
2
4
6
8
10
WT
Trehalose
Miglustat
JNCL

A
340/380
0.1
0
Time
(min)
1
2μM ionomycin
300μM GPN
control
CLN3
CLN3 +
miglustat

Figure 4

GPN evoked Ca2+ release
(340/380)
0.00
0.02
0.04
0.06
0.08
control
CLN3
CLN3 +
miglustat
*

Figure 4 (Continued)

B
CtxB
(ganglioside GM1)
Shiga-like toxin
(globoside Gb4)
SCMAS
Lysotracker
(lysosomes)
CytoID
(autophagy)
control
CLN3
CLN3 +
miglustat

C

Lysotracker (lysosomes)
Lysosomal area/cell
8000
6000
4000
2000
0
*
control
CLN3
CLN3 +
miglustat

CytoID (autophagy)
Percentage cells with
punctate staining
100
75
50
25
0
*
**
*
control
CLN3
CLN3 +
miglustat

C
CtxB (ganglioside GM1)
Percentage cells with punctate staining
0
25
50
75
100
control
CLN3
CLN3 + miglustat
*
Shiga-like toxin (globoside Gb4)
Percentage cells with punctate staining
0
25
50
75
100
control
CLN3
CLN3 + miglustat
*
SCMAS
Percentage cells with punctate staining
0
25
50
75
100
control
CLN3
CLN3 + miglustat
*

Figure 4 (Continued)

A

$Cln3^{+/+}$
NPC2
CtxB
merge
plot profile
— CtxB
— NPC2
$Cln3^{mutant}$
NPC2
CtxB
merge
plot profile
— CtxB
— NPC2
$Cln3^{mutant}$ + miglustat
NPC2
CtxB
merge
plot profile
— CtxB
— NPC2


Figure 5

B

X4000
mitochondria
lysosomes
X2500
Normal HF 1yr
Normal HF 54yr
CLN3 HF
CLN3 HF + miglustat
0.5 µm
0.5 µm

Figure 5 (Continued)

**Figure 6**

**A**

CtxB
(ganglioside GM1)
Shiga-like Toxin
(globoside Gb4)
SCMAS
Lysotracker
(lysosomes)
CytoID
(autophagy)
control
CLN3
CLN3 +
miglustat

**B**

Lysotracker (lysosomes)
Lysosomal area/cell
8000
6000
4000
2000
0
*
control
CLN3
CLN3 +
miglustat

CytoID (autophagy)
Percentage cells with
punctate staining
100
75
50
25
0
**
control
CLN3
CLN3 +
miglustat

B
CtxB (ganglioside GM1)
Percentage cells with punctate staining
0
25
50
75
100
**
control
CLN3
CLN3 + miglustat
Shiga-like toxin (globoside Gb4)
Percentage cells with punctate staining
0
25
50
75
100
*
control
CLN3
CLN3 + miglustat
SCMAS
Percentage cells with punctate staining
0
25
50
75
100
**
****
*
control
CLN3
CLN3 + miglustat

Figure 6 (Continued)

Figure 6 (Continued)

C

300μM GPN
2μM ionomycin
control
CLN3
CLN3 +
miglustat
0.1
340/380
0
1
Time
(min)
GPN evoked Ca2+ release
(340/380)
0.4
0.3
0.2
0.1
0.0
*
control
CLN3
CLN3 +
miglustat

2μM ionomycin
20μM glutamate
control
CLN3
CLN3 + miglustat
0.1
340/380
0
1
Time
(min)
Number of glutamate
induced release events
5
4
3
2
1
0
*
control
CLN3
CLN3 +
miglustat

Figure 7

A
uninjected
injection control
cln3 MO
cln3 MO + miglustat
CtxB (ganglioside GM1)
SCMAS
B
Normalised retinal area (% uninjected)
125
100
75
50
25
0
***
uninjected
injection control
cln3 MO
cln3 MO + miglustat

**Figure 7 (Continued)**

**C**

160
140
120
100
80
60
40
20
0
Mean grey
0
200
400
600
800
1000
Distance
injection control
cln3 MO
cln3 MO + miglustat

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017185010 A, Palmieri
- WO 2017136922 A
- US 20010033888 A
- EP 1896083 A


### Non-patent literature cited in the description


- **PLATT et al.** *JBC*, 1994
- **WAGER et al.** *PLOS One*, 2014
- **ABERG L** ; **AUTTI T** ; **BRAULKE T** ; **COOPER JD** ; **VAN DIGGELEN OP** ; **JALANKO A** ; **KENRICK S** ; **KITZMÜLLER C** ; **KOHLSCHÜTTER A** ; **KYTTÄLÄ A**. The Neuronal Ceroid Lipofuscinoses (Batten Disease). Oxford University Press, 2011, 110-139
- **BOZORG, S** ; **RAMIREZ-MONTEALEGRE, D** ; **CHUNG, M** ; **PEARCE, D.A.** Juvenile neuronal ceroid lipofuscinosis (JNCL) and the eye.. *Survey of ophthalmology*, 2009, vol. 54, 463-471
- **BUTTERS TD**. Pharmacotherapeutic strategies using small molecules for the treatment of glycolipid lysosomal storage disorders. *Expert Opin Pharmacother.*, 2007, vol. 8 (4), 427-35
- **CAO, Y** ; **ESPINOLA, J.A.** ; **FOSSALE, E** ; **MASSEY, A.C** ; **CUERVO, A.M** ; **MACDONALD, M.E.** ; **COTMAN, S.L.** Autophagy is disrupted in a knock-in mouse model of juvenile neuronal ceroid lipofuscinosis.. *J. Biol. Chem.*, 2006, vol. 281, 20483-20493
- **CÁRCEL-TRULLOLS, J.** ; **KOVÁCS, A.D** ; **PEARCE, D.A.** Cell biology of the NCL proteins. What they do and don't do.. *Biochimica et biophysica acta*, 2015, vol. 1852, 2242-2255
- **CENDRET, V. et al.** Synthetic deoxynojirimycin derivatives bearing a thiolated, fluorinated or unsaturated N-alkyl chain: identification of potent α-glucosidase and trehalase inhibitors as well as F508del-CFTR correctors.. *Org Biomol Chem.*, 21 November 2015, vol. 13 (43)
- **CHANDRACHUD U** ; **WALKER MW** ; **SIMAS AM** ; **HEETVELD S** ; **PETCHERSKI A** ; **KLEIN M** ; **OH H** ; **WOLF P** ; **ZHAO WN** ; **NORTON S**. Unbiased Cell-based Screening in a Neuronal Cell Model of Batten Disease Highlights an Interaction between Ca2+ Homeostasis, Autophagy, and CLN3 Protein Function.. *J Biol Chem.*, 2015, vol. 290 (23), 14361-80
- **DEBOSCH, B.J** ; **HEITMEIER, M.R** ; **MAYER, A.L** ; **HIGGINS, C.B** ; **CROWLEY, J.R** ; **KRAFT, T.E** ; **CHI, M.** ; **NEWBERRY, E.P** ; **CHEN, Z** ; **FINCK, B.N et al.** Trehalose inhibits solute carrier 2A (SLC2A) proteins to induce autophagy and prevent hepatic steatosis.. *Science signaling*, 2016, vol. 9, ra21
- **DEHAY, B.** ; **BOVÉ, J.** ; **RODRÍGUEZ-MUELA, N** ; **PERIER, C** ; **RECASENS, A** ; **BOYA, P.** ; **VILA, M.** Pathogenic lysosomal depletion in Parkinson's disease. *J.Neurosci.*, 2010, vol. 30, 12535-12544
- **DU, J** ; **LIANG, Y** ; **XU, F.** ; **SUN, B.** ; **WANG, Z.** Trehalose rescues Alzheimer's disease phenotypes in APP/PS1 transgenic mice.. *The Journal of pharmacy and pharmacology*, 2013, vol. 65, 1753-1756
- **FOSSALE, E** ; **WOLF, P.** ; **ESPINOLA, J.A.** ; **LUBICZ-NAWROCKA, T** ; **TEED, A.M** ; **GAO, H** ; **RIGAMONTI, D.** ; **CATTANEO, E** ; **MACDONALD, M.E** ; **COTMAN, S.L**. Membrane trafficking and mitochondrial abnormalities precede subunit c deposition in a cerebellar cell model of juvenile neuronal ceroid lipofuscinosis. *BMC.Neurosci.*, 2004, vol. 5, 57
- **GETTY, A.L** ; **PEARCE, D.A.** Interactions of the proteins of neuronal ceroid lipofuscinosis: clues to function. *Cell. Mol. Life Sci.*, 2011, vol. 68, 453-474
- **GROH, J.** ; **BERVE, K.** ; **MARTINI, R**. Fingolimod and Teriflunomide Attenuate Neurodegeneration in Mouse Models of Neuronal Ceroid Lipofuscinosis. *Molecular therapy : the journal of the American Society of Gene Therapy*, 2017, vol. 25, 1889-1899
- **JENNEMANN R. et al.** Cell-specific deletion of glucosylceramide synthase in brain leads to severe neural defects after birth.. *Proc Natl Acad Sci U S A.*, 30 August 2005, vol. 102 (35), 12459-64
- **KANG, S** ; **HEO, T.-H.** ; **KIM, S.-J.** Altered levels of α-synuclein and sphingolipids in Batten disease lymphoblast cells.. *Gene*, 2014, vol. 539, 181-185

- **KEMP P.J. et al.** Improving and accelerating the differentiation and functional maturation of human stem cell-derived neurons: role of extracellular calcium and GABA. *J Physiol.*, 15 November 2016, vol. 594 (22), 6583-6594
- **KHALIFEH, M** ; **BARRETO, G.E.** ; **SAHEBKAR, A.** Trehalose as a promising therapeutic candidate for the treatment of Parkinson's disease.. *Br J Pharmacol*, 2019, vol. 176, 1173-1189
- **LEE, HJ.** ; **YOON, YS.** ; **LEE, SJ**. Mechanism of neuroprotection by trehalose: controversy surrounding autophagy induction. *Cell Death Dis*, 2018, vol. 9, 712
- **LUIRO, K** ; **YLIANNALA, K** ; **AHTIAINEN, L.** ; **MAUNU, H** ; **JÄRVELÄ, I.** ; **KYTTÄLÄ, A** ; **JALANKO, A.** Interconnections of CLN3, Hook1 and Rab proteins link Batten disease to defects in the endocytic pathway. *Hum Mol.Genet.*, 2004, vol. 13, 3017-3027
- **MEDINA, D.L** ; **FRALDI, A** ; **BOUCHE, V** ; **ANNUNZIATA, F** ; **MANSUETO, G** ; **SPAMPANATO, C** ; **PURI, C** ; **PIGNATA, A** ; **MARTINA, J.A.** ; **SARDIELLO, M et al.** Transcriptional activation of lysosomal exocytosis promotes cellular clearance.. *Developmental cell*, 2011, vol. 21, 421-430
- **NIKOLETOPOULOU, V** ; **PAPANDREOU, M.-E** ; **TAVERNARAKIS, N.** Autophagy in the physiology and pathology of the central nervous system. *Cell death and differentiation*, 2015, vol. 22, 398-407
- **NIXON, R.A.** ; **YANG, D.-S.** Autophagy and neuronal cell death in neurological disorders. *Cold Spring Harbor perspectives in biology*, 2012, vol. 4
- **OSTERGAARD, J.R.** Juvenile neuronal ceroid lipofuscinosis (Batten disease). Current insights.. *Degenerative neurological and neuromuscular disease*, 2016, vol. 6, 73-83
- **PALMIERI, M.** ; **PAL, R** ; **NELVAGAL, H.R** ; **LOTFI, P** ; **STINNETT, G.R** ; **SEYMOUR, M.L** ; **CHAUDHURY, A.** ; **BAJAJ, L** ; **BONDAR, V.V.** ; **BREMNER, L et al.** mTORC1-independent TFEB activation via Akt inhibition promotes cellular clearance in neurodegenerative storage diseases. *Nat. Commun*, 2007, vol. 8, 14338
- **PALMIERI, M.** ; **PAL, R** ; **NELVAGAL, H.R** ; **LOTFI, P** ; **STINNETT, G.R** ; **SEYMOUR, M.L** ; **CHAUDHURY, A** ; **BAJAJ, L** ; **BONDAR, V.V** ; **BREMNER, L. et al.** Corrigendum: mTORC1-independent TFEB activation via Akt inhibition promotes cellular clearance in neurodegenerative storage diseases. *Nat. Commun*, 2017, vol. 8, 14338
- **PERUCHO, J.** ; **CASAREJOS, M.J** ; **GOMEZ, A** ; **SOLANO, R.M** ; **YÉBENES, J.G. DE** ; **MENA, M.A.** Trehalose protects from aggravation of amyloid pathology induced by isoflurane anesthesia in APP(swe) mutant mice. *Curr Alzheimer Res*, 2012, vol. 9, 334-343
- **PLATT FM. et al.** N-butyldeoxygalactonojirimycin inhibits glycolipid biosynthesis but does not affect N-linked oligosaccharide processing.. *J Biol Chem.*, 28 October 1994, vol. 269 (43), 27108-14
- **PLATT FM et al.** Substrate reduction therapy. *Acta Paediatr*, April 2008, vol. 97 (457), 88-93
- **PONTIKIS, C.C.** ; **CELLA, C.V** ; **PARIHAR, N** ; **LIM, M.J** ; **CHAKRABARTI, S** ; **MITCHISON, H.M** ; **MOBLEY, W.C** ; **REZAIE, P.** ; **PEARCE, D.A.** ; **COOPER, J.D.** Late onset neurodegeneration in the Cln3-/- mouse model of juvenile neuronal ceroid lipofuscinosis is preceded by low level glial activation.. *Brain research*, 2004, vol. 1023, 231-242
- **PORTBURY, S.D.** ; **HARE, D.J.** ; **SGAMBELLONI, C** ; **PERRONNES, K.** ; **PORTBURY, A.J.** ; **FINKELSTEIN, D.I.** ; **ADLARD, P.A.** Trehalose Improves Cognition in the Transgenic Tg2576 Mouse Model of Alzheimer's Disease.. *Journal of Alzheimer's disease : JAD*, 2017, vol. 60, 549-560
- **RADKE, J.** ; **KOLL, R** ; **GILL, E.** ; **WIESE, L.** ; **SCHULZ, A** ; **KOHLSCHÜTTER, A** ; **SCHUELKE, M** ; **HAGEL, C** ; **STENZEL, W** ; **GOEBEL, H.H.** Autophagic vacuolar myopathy is a common feature of CLN3 disease.. *Annals of clinical and translational neurology*, 2018, vol. 5, 1385-1393
- **RYCKMAN AE et al.** Metabolism of Glycosphingolipids and Their Role in the Pathophysiology of Lysosomal Storage Disorders.. *Int J Mol Sci.*, September 2020, vol. 21 (18), 6881
- **SARDIELLO, M** ; **PALMIERI, M.** ; **DI RONZA, A** ; **MEDINA, D.L** ; **VALENZA, M** ; **GENNARINO, V.A** ; **DI MALTA, C** ; **DONAUDY, F.** ; **EMBRIONE, V** ; **POLISHCHUK, R.S. et al.** A gene network regulating lysosomal biogenesis and function.. *Science*, 2009, vol. 325, 473-477
- **SARKAR, S.** ; **RUBINSZTEIN, D.C**. Huntington's disease. Degradation of mutant huntingtin by autophagy.. *FEBS J*, 2008, vol. 275, 4263-4270
- **SARKAR, S** ; **CHIGURUPATI, S** ; **RAYMICK, J** ; **MANN, D** ; **BOWYER, J.F.** ; **SCHMITT, T** ; **BEGER, R.D** ; **HANIG, J.P** ; **SCHMUED, L.C** ; **PAULE, M.G.** Neuroprotective effect of the chemical chaperone, trehalose in a chronic MPTP-induced Parkinson's disease mouse model.. *Neurotoxicology*, 2014, vol. 44, 250-262
- **SARKAR, S.** ; **DAVIES, J.E** ; **HUANG, Z** ; **TUNNACLIFFE, A** ; **RUBINSZTEIN, D.C**. Trehalose, a novel mTOR-independent autophagy enhancer, accelerates the clearance of mutant huntingtin and alpha-synuclein.. *J. Biol. Chem.*, 2007, vol. 282, 5641-5652

- **SCHMIDTKE, C** ; **TIEDE, S** ; **THELEN, M** ; **KÄKELÄ, R** ; **JABS, S** ; **MAKRYPIDI, G** ; **SYLVESTER, M.** ; **SCHWEIZER, M** ; **BRAREN, I.** ; **BROCKE-AHMADINEJAD, N. et al.** Lysosomal proteome analysis reveals that CLN3-defective cells have multiple enzyme deficiencies associated with changes in intracellular trafficking.. *J Biol Chem*, 2019, vol. 294, 9592-9604
- **SETTEMBRE, C** ; **DI MALTA, C** ; **POLITO, V.A** ; **GARCIA ARENCIBIA, M** ; **VETRINI, F** ; **ERDIN, S.** ; **ERDIN, S.U** ; **HUYNH, T.** ; **MEDINA, D** ; **COLELLA, P et al.** TFEB links autophagy to lysosomal biogenesis.. *Science*, 2011, vol. 332, 1429-1433
- **SOMOGYI A. et al.** Altered Expression of Ganglioside Metabolizing Enzymes Results in GM3 Ganglioside Accumulation in Cerebellar Cells of a Mouse Model of Juvenile Neuronal Ceroid Lipofuscinosis.. *Int J Mol Sci.*, 22 February 2018, vol. 19 (2), 625
- **SONG, W.** ; **WANG, F** ; **SAVINI, M** ; **AKE, A** ; **DI RONZA, A** ; **SARDIELLO, M** ; **SEGATORI, L.** TFEB regulates lysosomal proteostasis. *Human molecular genetics*, 2013, vol. 22, 1994-2009
- **TAGA, T. et al.** The crystal and molecular structure of trehalose dihydrate. *Acta Cryst*, 1972, vol. B28, 325
- **TANAKA, M** ; **MACHIDA, Y** ; **NIU, S** ; **IKEDA, T** ; **JANA, N.R** ; **DOI, H** ; **KUROSAWA, M** ; **NEKOOKI, M.** ; **NUKINA, N.** Trehalose alleviates polyglutamine-mediated pathology in a mouse model of Huntington disease.. *Nat. Med.*, 2004, vol. 10, 148-154
- **TE VRUCHTE D. et al.** Accumulation of glycosphingolipids in Niemann-Pick C disease disrupts endosomal transport.. *J Biol Chem.*, 18 June 2004, vol. 279 (25), 26167-75
- **TIEN, N.T.** ; **KARACA, I** ; **TAMBOLI, I.Y.** ; **WALTER, J.** Trehalose Alters Subcellular Trafficking and the Metabolism of the Alzheimer-associated Amyloid Precursor Protein. *J Biol Chem*, 2016, vol. 291, 10528-10540
- **UCHIDA, K.** ; **UNUMA, K** ; **FUNAKOSHI, T** ; **AKI, T** ; **UEMURA, K**. Activation of Master Autophagy Regulator TFEB During Systemic LPS Administration in the Cornea. *Journal of Toxicologic Pathology*, 2014, vol. 27, 153-158
- **UUSI-RAUVA, K** ; **LUIRO, K** ; **TANHUANPÄÄ, K** ; **KOPRA, O** ; **MARTÍN-VASALLO, P** ; **KYTTÄLÄ, A** ; **JALANKO, A.** Novel interactions of CLN3 protein link Batten disease to dysregulation of fodrin-Na+, K+ ATPase complex.. *Exp Cell Res*, 2008, vol. 314, 2895-2905
- **WADA S** ; **SAWA R** ; **OHBA S** ; **HAYASHI C** ; **UMEKITA M** ; **SHIBUYA Y** ; **IIJIMA K** ; **IWANAMI F** ; **IGARASHI M**. Stability and Bioavailability of Lentztrehaloses A, B, and C as Replacements for Trehalose.. *J Agric Food Chem.*, 2016, vol. 64 (38), 7121-6
- **WAGER K. et al.** Neurodegeneration and Epilepsy in a Zebrafish Model of CLN3 Disease (Batten Disease). *PLoS One.*, 21 June 2016, vol. 11 (6), e0157365
- **WEIMER, J. M.** ; **BENEDICT, J. W** ; **GETTY, A. L** ; **PONTIKIS, C. C.** ; **LIM, M. J** ; **COOPER, J. D** ; **PEARCE, D. A.** Cerebellar defects in a mouse model of juvenile neuronal ceroid lipofuscinosis. *Brain research*, 2009, vol. 1266, 93-107
- **ZHANG, X** ; **CHEN, S** ; **SONG, L.** ; **TANG, Y.** ; **SHEN, Y** ; **JIA, L.** ; **LE, W.** MTOR-independent, autophagic enhancer trehalose prolongs motor neuron survival and ameliorates the autophagic flux defect in a mouse model of amyotrophic lateral sclerosis. *Autophagy*, 2014, vol. 10, 588-602